# EUROPEAN PATENT SPECIFICATION

(11) **EP 3 394 617 B1**
(45) Date of publication and mention of the grant of the patent: **30.10.2024**
(21) Application number: 16879994.8
(22) Date of filing: 20.12.2016
(51) Int. Cl.: G01N 33/536, G01N 33/68

(54) **COMPOSITIONS, DEVICES, AND METHODS OF MIGRAINE HEADACHE FOOD SENSITIVITY TESTING**
ZUSAMMENSETZUNGEN, VORRICHTUNGEN UND VERFAHREN ZUM TESTEN DER LEBENSMITTELEMPFINDLICHKEIT BEI MIGRÄNE
COMPOSITIONS, DISPOSITIFS, ET MÉTHODES DE TEST DE SENSIBILITÉ ALIMENTAIRE INDUISANT LA CÉPHALÉE MIGRAINEUSE

(30) Priority: 21.12.2015 US 201562270582 P
(43) Date of publication of application: 31.10.2018
(73) Proprietor: Biomerica, Inc., Irvine, California 92614 (US)
(72) Inventor: IRANI-COHEN, Zackary, Irvine, California 92614 (US); LADERMAN, Elisabeth, Irvine, California 92614 (US)
(74) Representative: Schlich, George William
(86) International application number: PCT/US2016/067873
(87) International publication number: WO 2017/112707

(56) References cited:
- US-A1- 2004 072 272
- US-A1- 2007 122 840
- US-B2- 7 601 509
- QIANG ZENG ET AL: "Variable Food-Specific IgG Antibody Levels in Healthy and Symptomatic Chinese Adults", PLOS ONE, vol. 8, no. 1, 1 January 2013 (2013-01-01), pages 1 - 9, XP055367030, DOI: 10.1371/journal.pone.0053612
- VASILIOS CONSTANTINIDES ET AL: "Migraine and tension-type headache triggers in a Greek population", ARQUIVOS DE NEURO-PSIQUIATRIA, vol. 73, no. 8, 1 August 2015 (2015-08-01), pages 665 - 669, XP055596996, DOI: 10.1590/0004-282X20150093
- ZENG , QIANG ET AL.: "Variable food-specific IgG antibody levels in healthy and symptomatic Chinese adults", PLOS ONE, vol. 8, no. 1, 2013, pages e53612, XP055367030
- ALPAY , KADRIYE ET AL.: "Diet restriction in migraine, based on IgG against foods: A clinical double-blind, randomised, cross-over trial", CEPHALALGIA, vol. 30, no. 7, 2010, pages 829 - 837, XP055395419
- MITCHELL , NATASHA ET AL.: "Randomised controlled trial of food elimination diet based on IgG antibodies for the prevention of migraine like headaches", NUTRITION JOURNAL, vol. 10, no. 85, 2011, pages 1 - 9, XP021105443

## Description

### Field

Sensitivity testing for food intolerance as it relates to the testing and possible elimination of selected food items as trigger foods for patients diagnosed with or suspected to have migraine headaches are described herein.

### Background

The background description includes information that may be useful in understanding the present disclosure. It is not an admission that any of the information provided herein is prior art or relevant to the appended claims, or that any publication specifically or implicitly referenced is prior art.

Food sensitivity (also known as food intolerance), especially as it relates to migraine headache (a type of chronic neurological disease), often presents with pain, nausea, vomiting, sensitivity to light, sound, or smell and underlying causes of migraine headaches are not well understood in the medical community. Most typically, migraine headaches are diagnosed by signs, symptoms along with neuroimaging tests. Unfortunately, treatments of migraine headaches are often less than effective and may present new difficulties due to neuromodulatory effects. Elimination of other one or more food items may be useful in at least reducing incidence and/or severity of the symptoms. However, migraine headaches are often quite diverse with respect to dietary items triggering symptoms, and no standardized test to help identify trigger food items with a reasonable degree of certainty is known, leaving such patients often to trial-and-error.

While there are some commercially available tests and labs to help identify trigger foods, the quality of the test results from these labs is generally poor as is reported by a consumer advocacy group (*e.g*., http://www.which.co.uk/news/2008/08/food-allergy-tests-could-risk-your-health-154711/). Most notably, problems associated with these tests and labs were high false positive rates, high intra-patient variability, and inter-laboratory variability, rendering such tests nearly useless. Similarly, further inconclusive and highly variable test results were also reported elsewhere (Alternative Medicine Review, Vol. 9, No. 2, 2004: pp 198-207), and the authors concluded that this may be due to food reactions and food sensitivities occurring via a number of different mechanisms. For example, not all migraine headache patients show positive response to food A, and not all migraine headache patients show negative response to food B. Thus, even if a migraine headache patient shows positive response to food A, removal of food A from the patient's diet may not relieve the patient's migraine headache symptoms. In other words, it is not well determined whether food allergens used in the currently available tests are properly selected based on high probabilities of correlating sensitivities to those food allergens to migraine headache.

In US 7,601,509 B2, there is disclosed, according to its abstract, "a diet-typing system for humans, including novel methods for diagnosis and treatment of food allergies and hypersensitivities".

In a publication in PLOS ONE by Qiang Zeng et al from January 2013, there is disclosed, according to its title, "Variable Food-Specific IgG Antibody Levels in Healthy and Symptomatic Chinese Adults".

In a publication in Arquivos de Neuro-Psiquiatria by Constantinides et al from August 2015, there is disclosed, according to its abstract, a study "to detect differences in clinical characteristics and headache triggers and in a Greek cohort of 51 migraineurs and 12 patients with tension-type headache".

Thus, even though various tests for food sensitivities are known in the art, all or almost all of them suffer from one or more disadvantages. Therefore, there is still a need for improved compositions, devices, and methods of food sensitivity testing, especially for identification and possible elimination of trigger foods for patients identified with or suspected of having migraine headaches.

### Summary

The present invention provides a test panel for testing for food intolerance in a patient diagnosed with, or suspected of having, migraine headaches, the test panel comprising:
a plurality of distinct food preparations, wherein each food preparation is independently coupled to an individually addressable solid carrier;
wherein the plurality of distinct food preparations consists of cucumber, tomato, malt, cauliflower, broccoli, peach, cantaloupe, orange, egg, tea, cabbage, green pepper, safflower, grapefruit, Swiss cheese, chocolate, wheat, cow's milk, rye, baker's yeast, cottage cheese, brewer's yeast, oat, honey, almond, sweet potato, onion, lemon, cheddar cheese, butter, rice, cane sugar, parsley, mustard, tobacco, goat's milk, American cheese, yogurt, eggplant, black walnut, spinach, cola nut, avocado, corn, garlic, pineapple, strawberry, sunflower seed, buck wheat, beef, potato, and mushroom.

The plurality of distinct food preparations have an average discriminatory p-value of ≤ 0.07 as determined by raw p-value and an average discriminatory p-value of ≤ 0.10 as determined by FOR multiplicity adjusted p-value. In certain embodiments, the average discriminatory p-value is determined by comparing assay values of a first patient test cohort that is diagnosed with or suspected of having migraine headaches with assay values of a second patient test cohort that is not diagnosed with or suspected of having migraine headaches. In such embodiments, the assay values can be determined by conducting assays for the first and second patient test cohorts with the distinct food preparation.

The distinct food preparations may be crude filtered aqueous extracts or processed aqueous extracts.

The solid carrier may be a well of a multiwell plate, a bead, an electrical sensor, a chemical sensor, a microchip or an adsorptive film.

The present invention also provides an *in vitro* method of testing food intolerance in patients that are diagnosed with or suspected to have migraine headaches, comprising:
contacting a food preparation, said food preparation having at least one component, with a bodily fluid of a patient that is diagnosed with or suspected of having migraine headaches, wherein the bodily fluid comprises at least one immunoglobulin, wherein the bodily fluid is associated with a gender identification, and wherein the contacting is performed under conditions that allow at least a portion of the immunoglobulin to bind to the at least one component;
measuring the portion of the immunoglobulin bound to the at least one component of the food preparation to obtain a signal;
comparing the signal to a gender-stratified reference value for the food preparation using the gender identification to obtain a result; and
updating or generating a report using the result,
wherein the contacting a food preparation is performed with a multiplexed assay comprising a plurality of distinct food preparations; and
wherein the plurality of distinct food preparations consists of cucumber, tomato, malt, cauliflower, broccoli, peach, cantaloupe, orange, egg, tea, cabbage, green pepper, safflower, grapefruit, Swiss cheese, chocolate, wheat, cow's milk, rye, baker's yeast, cottage cheese, brewer's yeast, oat, honey, almond, sweet potato, onion, lemon, cheddar cheese, butter, rice, cane sugar, parsley, mustard, tobacco, goat's milk, American cheese, yogurt, eggplant, black walnut, spinach, cola nut, avocado, corn, garlic, pineapple, strawberry, sunflower seed, buck wheat, beef, potato, and mushroom.

The bodily fluid of the patient may comprise whole blood, plasma, serum, saliva, or a fecal suspension.

The gender-stratified reference value for each of the food preparations may be the 90^{th} percentile rank, or higher, of signals obtained by contacting bodily fluid from a control group of subjects that is not diagnosed with or suspected of having migraine headaches with the food preparation.

Various objects, features, aspects and advantages of the embodiments described herein will become more apparent from the following detailed description of preferred embodiments, along with the accompanying drawing figures in which like numerals represent like components.

### Brief Description of The Drawings and Tables

**Table 1** shows a list of food items from which food preparations can be prepared.
**Table 2** shows statistical data of foods ranked according to 2-tailed FDR multiplicity-adjusted p-values.
**Table 3** shows statistical data of ELISA score by food and gender.
**Table 4** shows cutpoint values of foods for a predetermined percentile rank.
**Figure 1A** illustrates ELISA signal score of male migraine headache patients and control tested with cucumber.
**Figure 1B** illustrates a distribution of percentage of male migraine headache subjects exceeding the 90^{th} and 95^{th} percentile tested with cucumber.
**Figure 1C** illustrates a signal distribution in women along with the 95^{th} percentile cutoff as determined from the female control population tested with cucumber.
**Figure 1D** illustrates a distribution of percentage of female migraine headache subjects exceeding the 90^{th} and 95^{th} percentile tested with cucumber.
**Figure 2A** illustrates ELISA signal score of male migraine headache patients and control tested with tomato.
**Figure 2B** illustrates a distribution of percentage of male migraine headache subjects exceeding the 90^{th} and 95^{th} percentile tested with tomato.
**Figure 2C** illustrates a signal distribution in women along with the 95^{th} percentile cutoff as determined from the female control population tested with tomato.
**Figure 2D** illustrates a distribution of percentage of female migraine headache subjects exceeding the 90^{th} and 95^{th} percentile tested with tomato.
**Figure 3A** illustrates ELISA signal score of male migraine headaches patients and control tested with malt.
**Figure 3B** illustrates a distribution of percentage of male migraine headaches subjects exceeding the 90^{th} and 95^{th} percentile tested with malt.
**Figure 3C** illustrates a signal distribution in women along with the 95^{th} percentile cutoff as determined from the female control population tested with malt.
**Figure 3D** illustrates a distribution of percentage of female migraine headaches subjects exceeding the 90^{th} and 95^{th} percentile tested with malt.
**Figure 4A** illustrates ELISA signal score of male migraine headaches patients and control tested with cauliflower.
**Figure 4B** illustrates a distribution of percentage of male migraine headaches subjects exceeding the 90^{th} and 95^{th} percentile tested with cauliflower.
**Figure 4C** illustrates a signal distribution in women along with the 95^{th} percentile cutoff as determined from the female control population tested with cauliflower.
**Figure 4D** illustrates a distribution of percentage of female migraine headache subjects exceeding the 90^{th} and 95^{th} percentile tested with cauliflower.
**Figures 5A** illustrates distributions of migraine headache subjects by number of foods that were identified as trigger foods at the 90^{th} percentile.
**Figures 5B** illustrates distributions of migraine headache subjects by number of foods that were identified as trigger foods at the 95^{th} percentile.
**Table 5A** shows raw data of migraine headache patients and control with number of positive results based on the 90^{th} percentile.
**Table 5B** shows raw data of migraine headache patients and control with number of positive results based on the 95^{th} percentile.
**Table 6A** shows statistical data summarizing the raw data of migraine headache patient populations shown in Table 5A.
**Table 6B** shows statistical data summarizing the raw data of migraine headache patient populations shown in Table 5B.
**Table 7A** shows statistical data summarizing the raw data of control populations shown in Table 5A.
**Table 7B** shows statistical data summarizing the raw data of control populations shown in Table 5B.
**Table 8A** shows statistical data summarizing the raw data of migraine headache patient populations shown in Table 5A transformed by logarithmic transformation.
**Table 8B** shows statistical data summarizing the raw data of migraine headache patient populations shown in Table 5B transformed by logarithmic transformation.
**Table 9A** shows statistical data summarizing the raw data of control populations shown in Table 5A transformed by logarithmic transformation.
**Table 9B** shows statistical data summarizing the raw data of control populations shown in Table 5B transformed by logarithmic transformation.
**Table 10A** shows statistical data of an independent T-test to compare the geometric mean number of positive foods between the migraine headache and non-migraine headache samples based on the 90^{th} percentile.
**Table 10B** shows statistical data of an independent T-test to compare the geometric mean number of positive foods between the migraine headache and non-migraine headache samples based on the 95^{th} percentile.
**Table 11A** shows statistical data of a Mann-Whitney test to compare the geometric mean number of positive foods between the migraine headache and non-migraine headache samples based on the 90^{th} percentile.
**Table 11B** shows statistical data of a Mann-Whitney test to compare the geometric mean number of positive foods between the migraine headache and non-migraine headache samples based on the 95^{th} percentile.

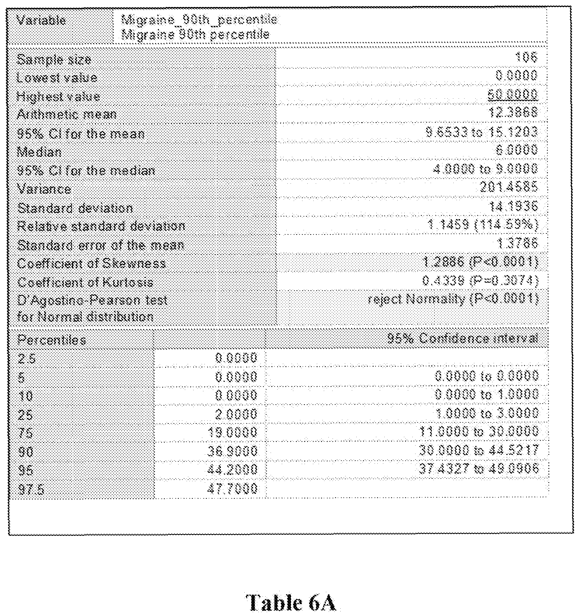

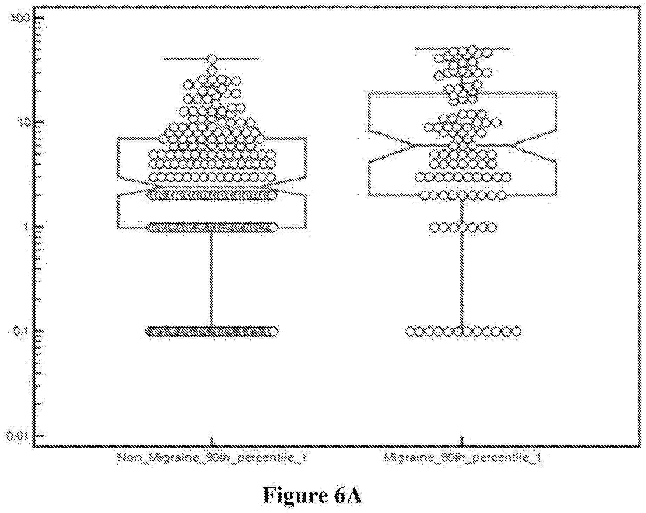

**Figure 6A** illustrates a box and whisker plot of data shown in Table 5A.

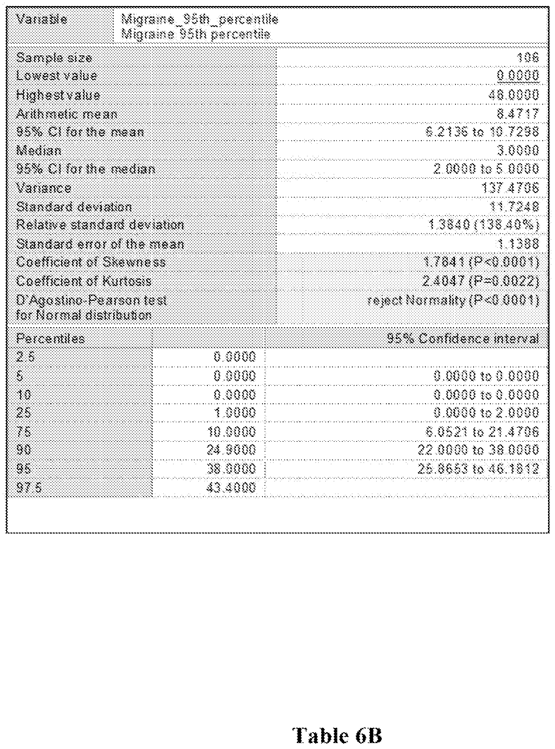

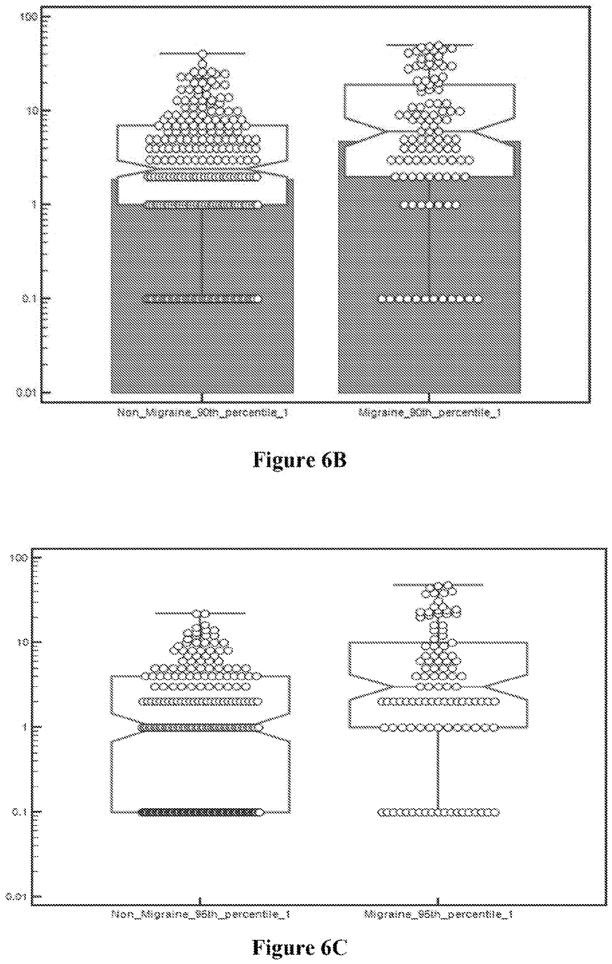

**Figure 6B** illustrates a notched box and whisker plot of data shown in Table 5A.
**Figure 6C** illustrates a box and whisker plot of data shown in Table 5B.

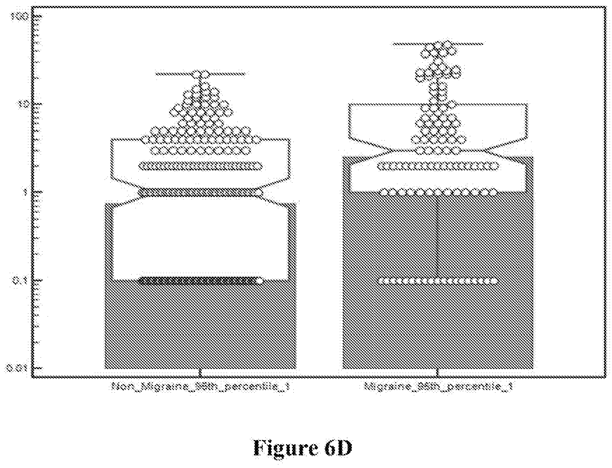

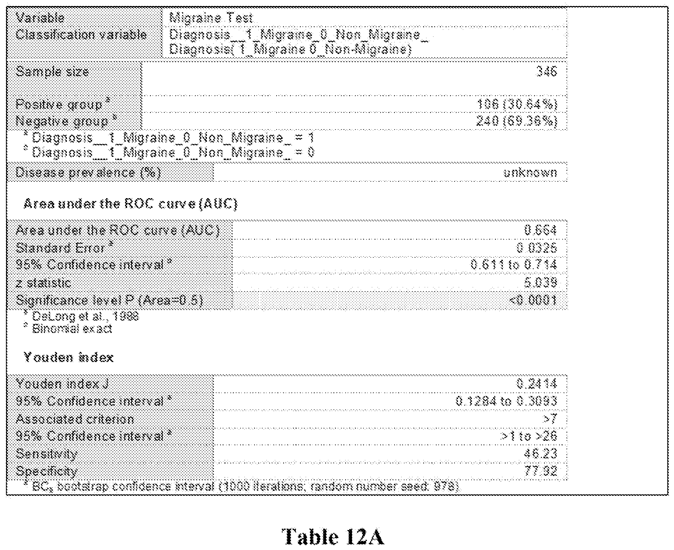

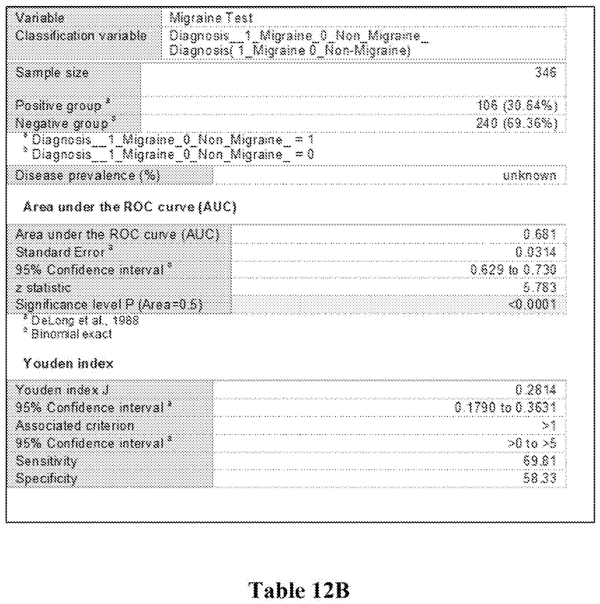

**Figure 6D** illustrates a notched box and whisker plot of data shown in Table 5B.
**Table 12A** shows statistical data of a Receiver Operating Characteristic (ROC) curve analysis of data shown in Tables 5A-11A.
**Table 12B** shows statistical data of a Receiver Operating Characteristic (ROC) curve analysis of data shown in Tables 5B-1 1B.

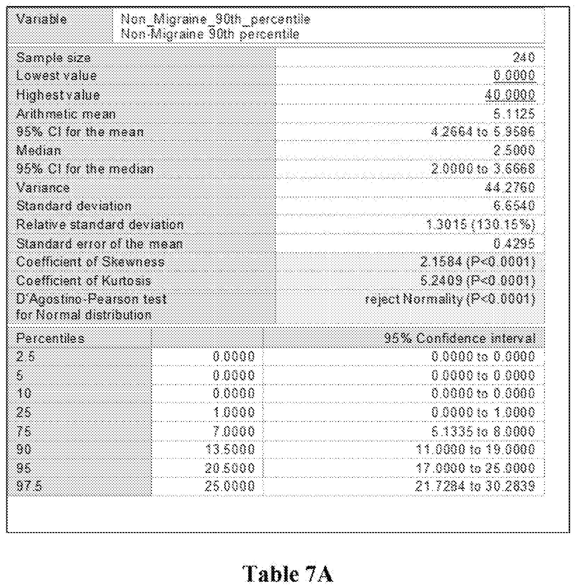

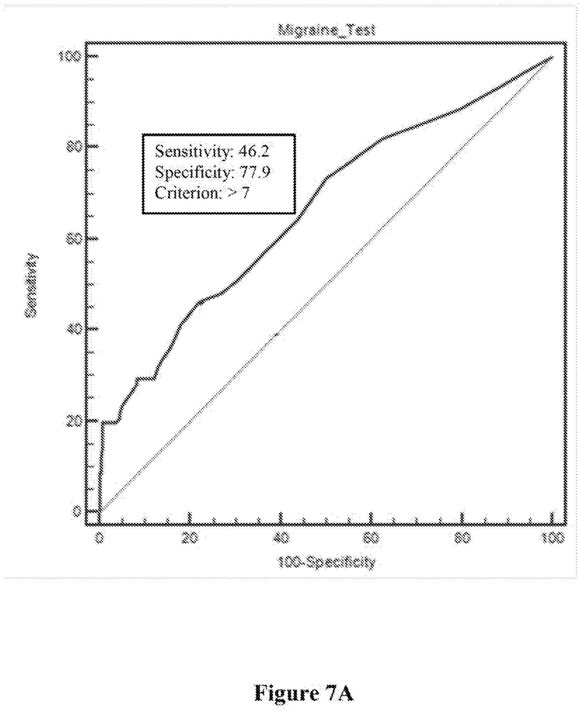

**Figure 7A** illustrates the ROC curve corresponding to the statistical data shown in Table 12A.

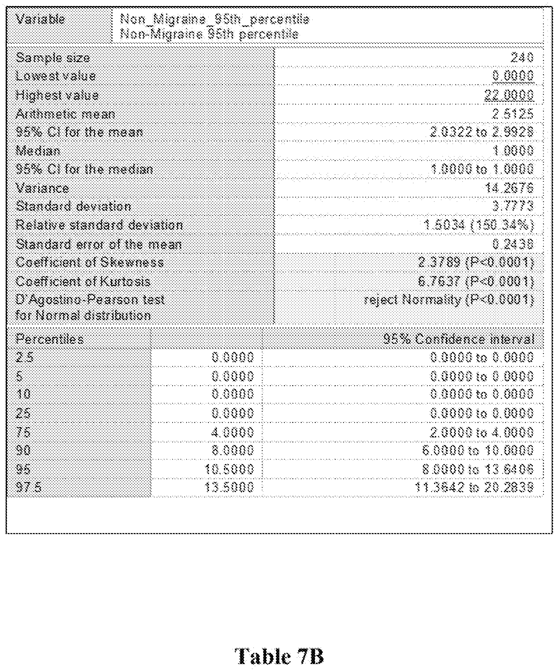

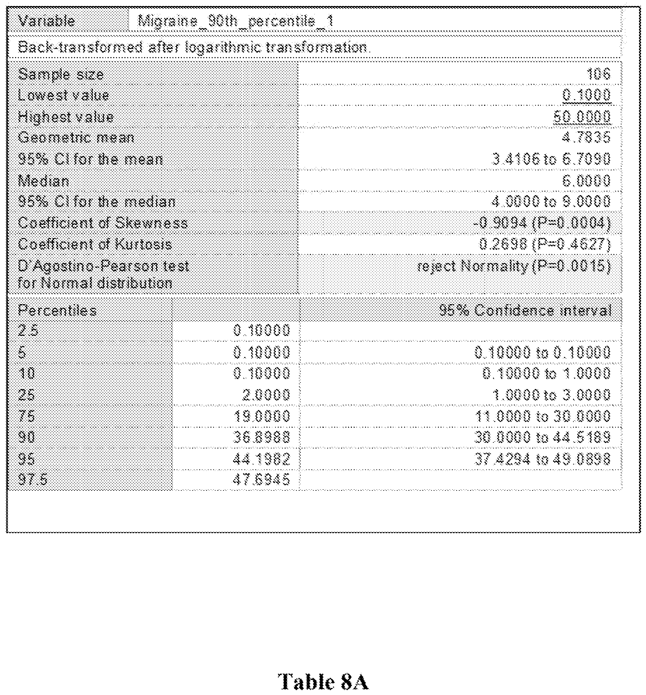

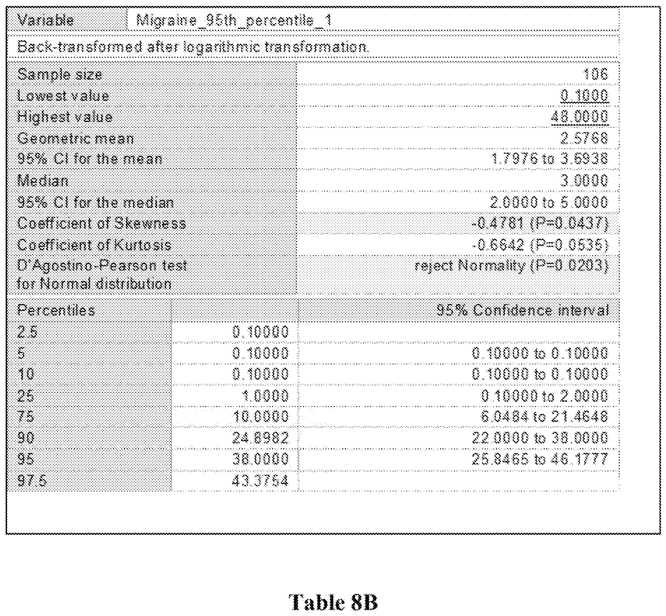

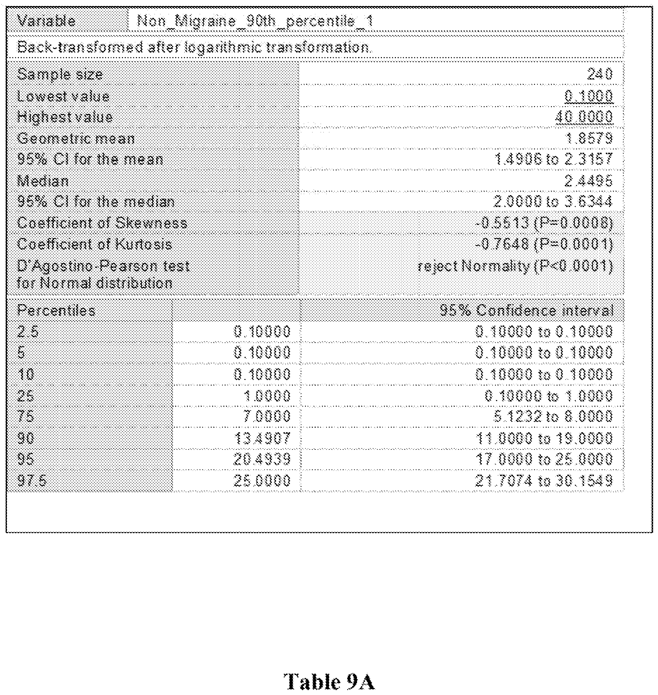

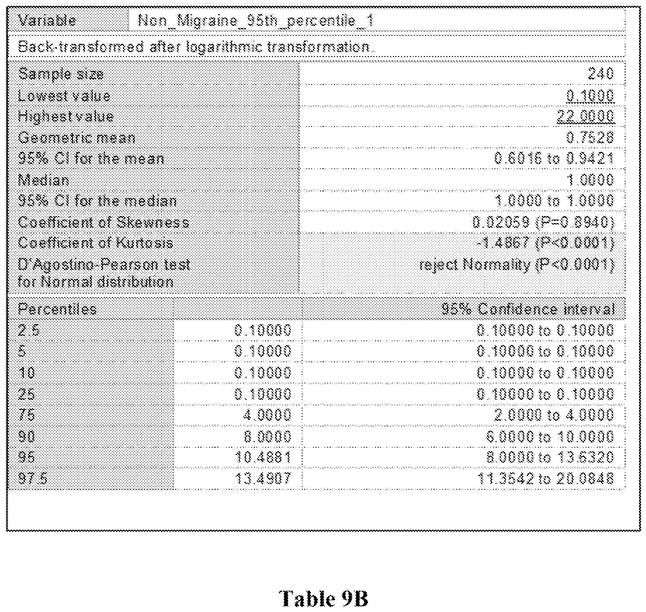

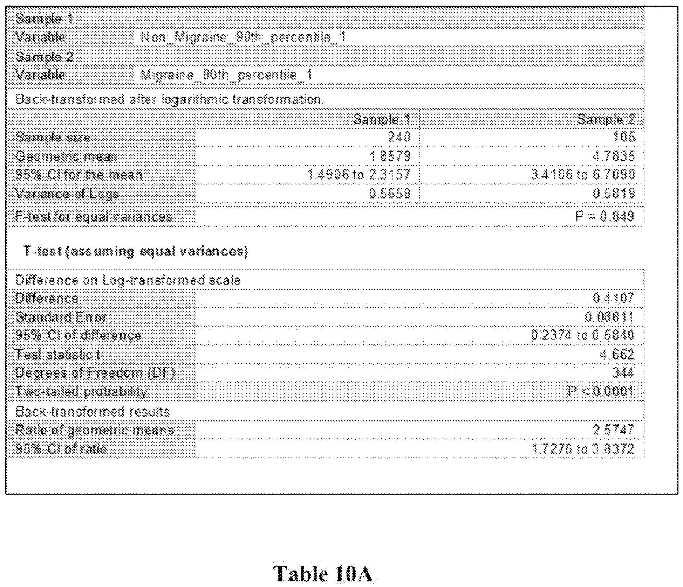

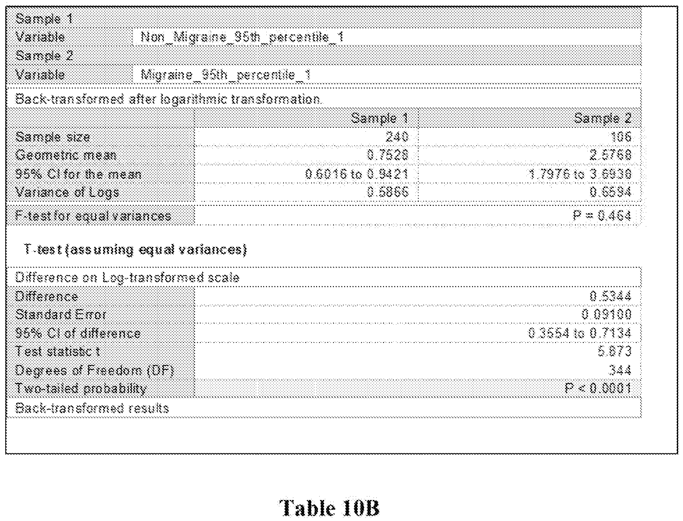

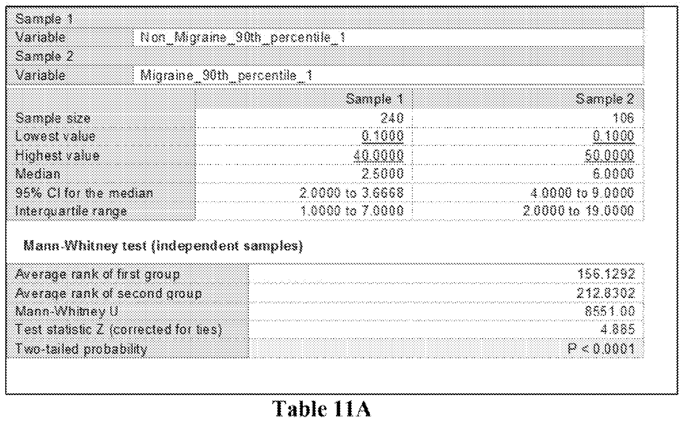

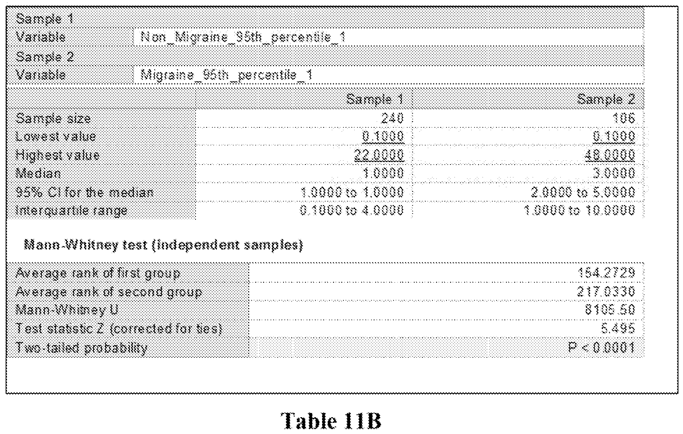

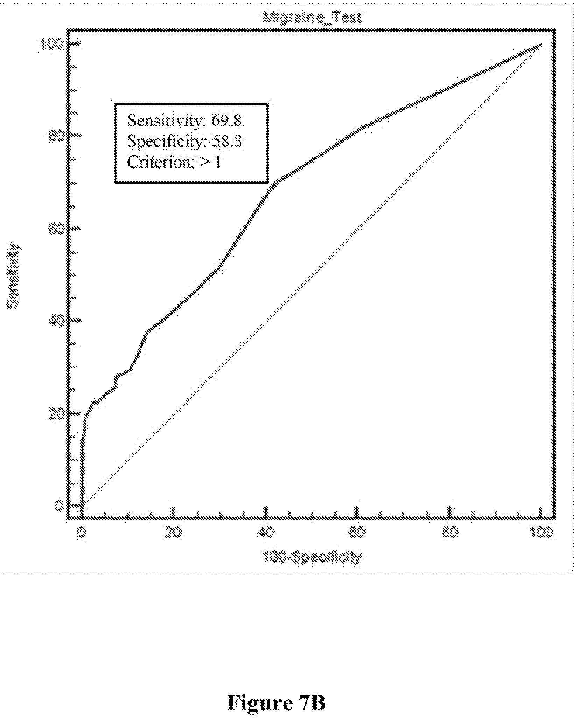

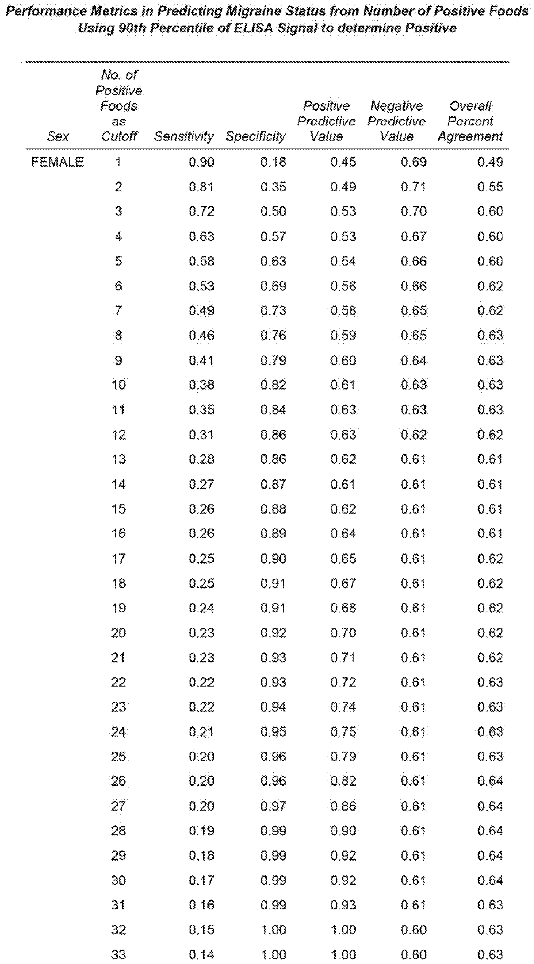

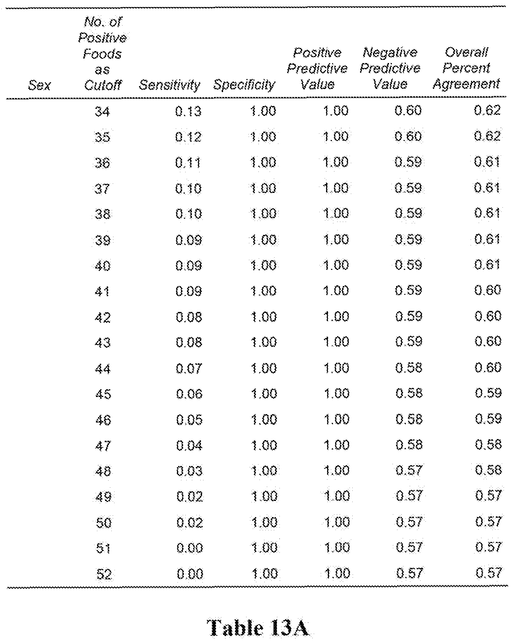

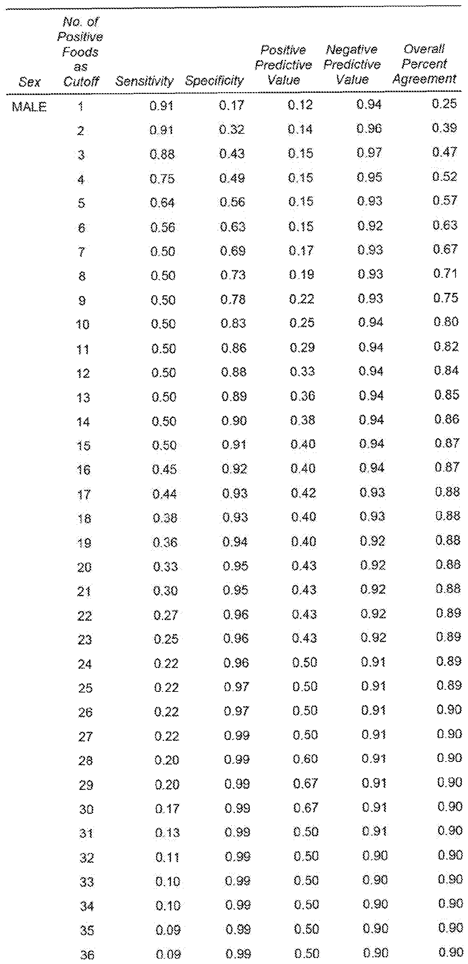

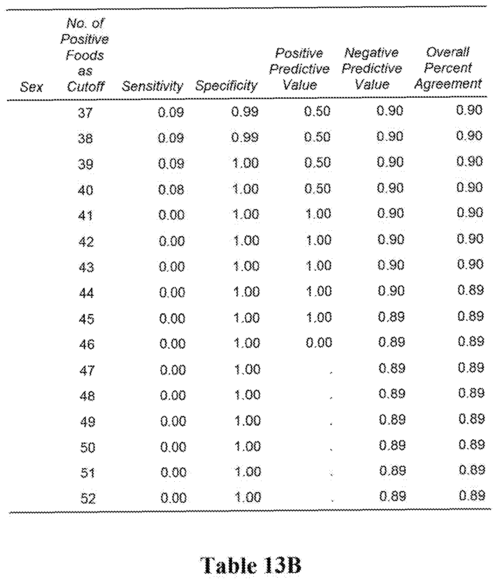

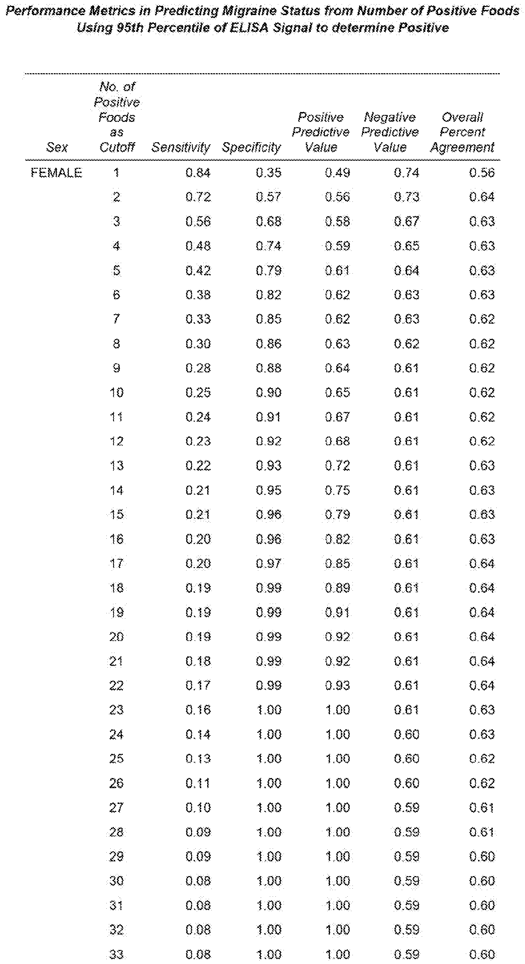

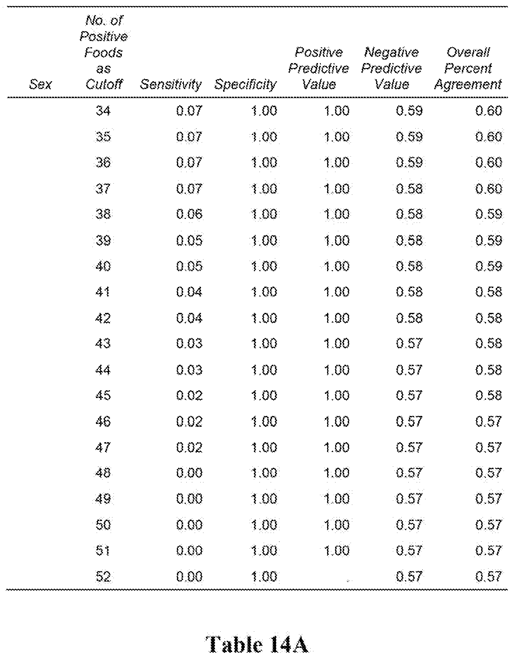

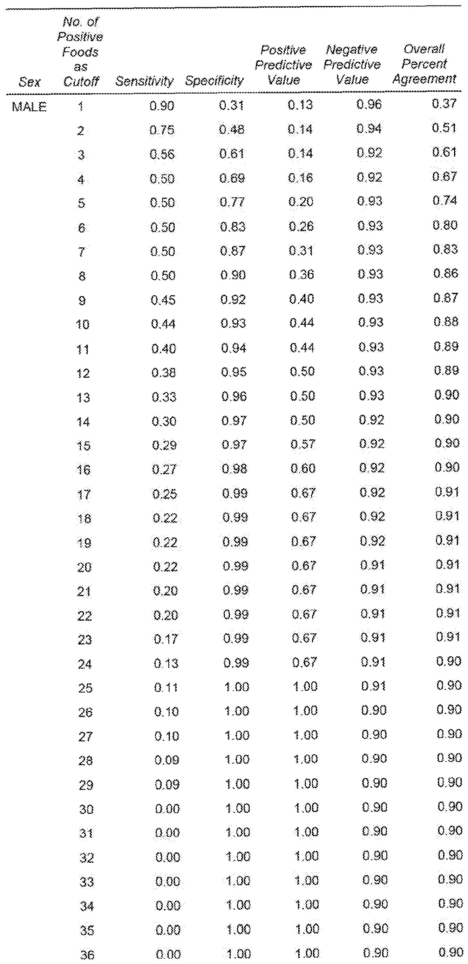

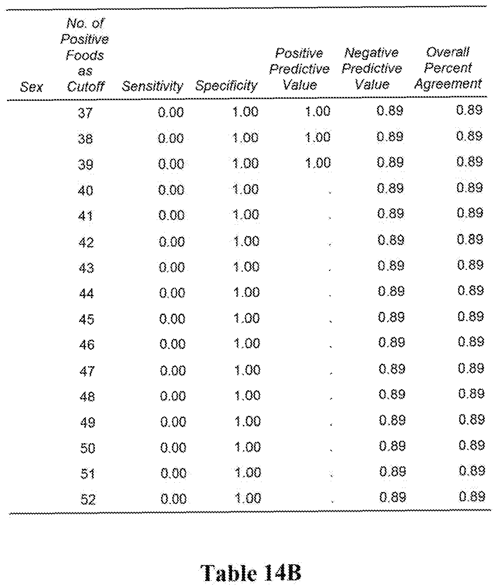

**Figure 7B** illustrates the ROC curve corresponding to the statistical data shown in Table 12B.
**Table 13A** shows a statistical data of performance metrics in predicting migraine headache status among female patients from number of positive foods based on the 90^{th} percent! le.
**Table 13B** shows a statistical data of performance metrics in predicting migraine headache status among male patients from number of positive foods based on the 90^{th} percentile.
**Table 14A** shows a statistical data of performance metrics in predicting migraine headache status among female patients from number of positive foods based on the 95^{th} percentile.
**Table 14B** shows a statistical data of performance metrics in predicting migraine headache status among male patients from number of positive foods based on the 95^{th} percentile.

### Detailed Description

The inventors have discovered that food preparations used in certain food tests to identify trigger foods in patients diagnosed with or suspected to have migraine headaches are not necessarily predictive of, or otherwise associated with, migraine headache symptoms. Indeed, various experiments have revealed that among a wide variety of food items, certain food items are highly predictive/associated with migraine headaches, whereas others may have no statistically significant association with migraine headaches.

Even more unexpectedly, the inventors discovered that in addition to the high variability of food items, gender variability with respect to response in a test may play a substantial role in the determination of association of a food item with migraine headaches. Consequently, based on the inventors' findings and further contemplations, test kits and methods are now presented with substantially higher predictive power in the choice of food items that could be eliminated for reduction of migraine headache signs and symptoms.

The following discussion provides many example embodiments of the inventive subject matter. Although each embodiment represents a single combination of inventive elements, the inventive subject matter is considered to include all possible combinations of the disclosed elements. Thus, if one embodiment comprises elements A, B, and C, and a second embodiment comprises elements B and D, then the inventive subject matter is also considered to include other remaining combinations of A, B, C, or D, even if not explicitly disclosed.

Food sensitivity (also known as food intolerance), especially as it relates to migraine headache (a type of chronic neurological disease), often presents with pain, nausea, vomiting, sensitivity to light, sound, or smell and underlying causes of migraine headaches are not well understood in the medical community. Most typically, migraine headaches are diagnosed by signs, symptoms along with neuroimaging tests. Unfortunately, treatments of migraine headaches are often less than effective and may present new difficulties due to neuromodulatory effects. Elimination of other one or more food items may be useful in at least reducing incidence and/or severity of the symptoms. However, migraine headaches are often quite diverse with respect to dietary items triggering symptoms, and no standardized test to help identify trigger food items with a reasonable degree of certainty is known, leaving such patients often to trial-and-error.

In some embodiments, the numbers expressing quantities or ranges, used to describe and claim certain embodiments of the disclosure are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the disclosure are approximations, the numerical values set forth in the specific examples are reported as precisely as practicable. The numerical values presented in some embodiments of the disclosure may contain certain errors necessarily resulting from the standard deviation found in their respective testing measurements. Unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

All methods described herein can be performed in any suitable order unless otherwise indicated herein or otherwise clearly contradicted by context. The use of any and all examples, or exemplary language (e.g., "such as") provided with respect to certain embodiments herein is intended merely to better illuminate the disclosure and does not pose a limitation on the scope of the disclosure otherwise claimed. No language in the specification should be construed as indicating any non-claimed element essential to the practice of the disclosure.

In some embodiments, the numbers expressing quantities of ingredients, properties such as concentration, reaction conditions, and so forth, used to describe and claim certain embodiments of the disclosure are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that, the numerical ranges and parameters setting forth the broad scope of some embodiments of the disclosure are approximations, the numerical values set forth in the specific examples are reported as precisely as practicable. The numerical values presented in some embodiments of the disclosure may contain certain errors necessarily resulting from the standard deviation found in their respective testing measurements. Moreover, and unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

While not limiting to the inventive subject matter, food preparations will typically be drawn from foods generally known or suspected to trigger signs or symptoms of migraine headaches. Particularly suitable food preparations may be identified by the experimental procedures outlined below.

The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the disclosure and does not pose a limitation on the scope of the disclosure otherwise claimed. No language in the specification should be construed as indicating any non-claimed element essential to the practice of the disclosure.

Of course, it should be noted that the particular format of the test kit or panel may vary considerably, and contemplated formats include micro well plates, dip sticks, membrane-bound arrays, etc. Consequently, the solid carrier to which the food preparations are coupled may include wells of a multiwell plate, a bead (*e.g*., color-coded or magnetic, etc.), an adsorptive film (*e.g*., nitrocellulose or micro/nanoporous polymeric film, etc.), or an electrical sensor (*e.g*. a printed copper sensor or microchip, etc.).

Consequently, the inventors also contemplate a method of testing food intolerance in patients that are diagnosed with or suspected to have migraine headaches. As also noted above, all of the different food preparations have an average discriminatory p-value of ≤ 0.07 as determined by raw p-value, and an average discriminatory p-value of ≤ 0.10 as determined by FDR multiplicity adjusted p-value.

While in certain embodiments food preparations are prepared from single food items as crude extracts, or crude filtered extracts, it is contemplated that food preparations can be prepared from mixtures of a plurality of food items (*e.g*. a mixture of citrus comprising lemon, orange, and a grapefruit, a mixture of yeast comprising baker's yeast and brewer's yeast, a mixture of rice comprising a brown rice and white rice, a mixture of sugars comprising honey, malt, and cane sugar. In some embodiments, it is also contemplated that food preparations can be prepared from purified food antigens or recombinant food antigens.

Each food preparation is immobilized on a solid surface in an addressable manner, such that each food preparation is isolated), it is contemplated that the step of measuring the IgG or other type of antibody bound to the component of the food preparation is performed via an ELISA (enzyme-linked immunosorbent assay) test. Exemplary solid surfaces include, but are not limited to, wells in a multiwell plate, such that each food preparation may be isolated to a separate microwell. In certain embodiments, the food preparation will be coupled to, or immobilized on, the solid surface.

Thus, it should be appreciated that by having a high-confidence test system as described herein, the rate of false-positive and false negatives can be significantly reduced, and especially where the test systems and methods are gender stratified or adjusted for gender differences as shown below. Such advantages have heretofore not been realized and it is expected that the systems and methods presented herein will substantially increase the predictive power of food sensitivity tests for patients diagnosed with or suspected to have migraine headaches.

### Experiments

General Protocol for food preparation generation: Commercially available food extracts (available from Biomerica Inc., 17571 Von Karman Ave, Irvine, CA 92614) prepared from the edible portion of the respective raw foods were used to prepare ELISA plates following the manufacturer's instructions.

For some food extracts, the inventors expect that food extracts prepared with specific procedures to generate food extracts may provides more superior results in detecting elevated IgG reactivity in migraine headache patients compared to commercially available food extracts. For example, for grains and nuts, a three-step procedure of generating food extracts may provide more accurate results. The first step is a defatting step. In this step, lipids from grains and nuts are extracted by contacting the flour of grains and nuts with a non-polar solvent and collecting residue. Then, the defatted grain or nut flour are extracted by contacting the flour with elevated pH to obtain a mixture and removing the solid from the mixture to obtain the liquid extract. Once the liquid extract is generated, the liquid extract is stabilized by adding an aqueous formulation. In one embodiment, the aqueous formulation includes a sugar alcohol, a metal chelating agent, protease inhibitor, mineral salt, and buffer component 20-50 mM of buffer from 4-9 pH. This formulation allowed for long term storage at -70 °C and multiple freeze-thaws without a loss of activity.

For another example, for meats and fish, a two-step procedure of generating food extract may provide more accurate results. The first step is an extraction step. In this step, extracts from raw, uncooked meats or fish are generated by emulsifying the raw, uncooked meats or fish in an aqueous buffer formulation in a high impact pressure processor. Then, solid materials are removed to obtain liquid extract. Once the liquid extract is generated, the liquid extract is stabilized by adding an aqueous formulation. In one embodiment, the aqueous formulation includes a sugar alcohol, a metal chelating agent, protease inhibitor, mineral salt, and buffer component 20-50 mM of buffer from 4-9 pH. This formulation allowed for long term storage at -70 °C and multiple freeze-thaws without a loss of activity.

For still another example, for fruits and vegetables, a two-step procedure of generating food extract is may provide more accurate results. The first step is an extraction step. In this step, liquid extracts from fruits or vegetables are generated using an extractor (e.g., masticating juicer, etc) to pulverize foods and extract juice. Then, solid materials are removed to obtain liquid extract. Once the liquid extract is generated, the liquid extract is stabilized by adding an aqueous formulation. In one embodiment, the aqueous formulation includes a sugar alcohol, a metal chelating agent, protease inhibitor, mineral salt, and buffer component 20-50 mM of buffer from 4-9 pH. This formulation allowed for long term storage at -70 °C and multiple freeze-thaws without a loss of activity.

Blocking of ELISA plates: To optimize signal to noise, plates will be blocked with a proprietary blocking buffer. In one embodiment, the blocking buffer includes 20-50 mM of buffer from 4-9 pH, a protein of animal origin (e.g., beef, chicken, etc.) and a short chain alcohol (e.g., glycerin, etc.). Other blocking buffers, including several commercial preparations, can be attempted but may not provide adequate signal to noise and low assay variability required.

ELISA preparation and sample testing: Food antigen preparations were immobilized onto respective microtiter wells following the manufacturer's instructions. For the assays (e.g., multiplexed assays, etc.), the food antigens were allowed to react with antibodies present in the patients' serum, and excess serum proteins were removed by a wash step. For detection of IgG antibody binding, enzyme labeled anti-IgG antibody conjugate was allowed to react with antigen-antibody complex. A color was developed by the addition of a substrate that reacts with the coupled enzyme. The color intensity was measured and is directly proportional to the concentration of IgG antibody specific to a particular food antigen.

Methodology to determine ranked food list in order of ability of ELISA signals to distinguish migraine headaches from control subjects: Out of an initial selection (e.g., 100 food items, or 150 food items, or even more), samples can be eliminated prior to analysis due to low consumption in an intended population. In addition, specific food items can be used as being representative of a larger more generic food group, especially where prior testing has established a correlation among different species within a generic group (with respect to both genders, or correlation with a single gender). For example, Swiss cheese could be dropped in favor of cheddar cheese as representative of the "cheese" food group. In further aspects, the final list foods will be shorter than 50 food items, or equal or less than of 40 food items.

Since the foods ultimately selected for the food intolerance panel will not be specific for a particular gender, in certain embodiments a gender-neutral food list is necessary. Since the observed sample will be at least initially imbalanced by gender (*e.g*., Controls: 50% female, migraine headaches: 87% female), differences in ELISA signal magnitude strictly due to gender will be removed by modeling signal scores against gender using a two-sample t-test and storing the residuals for further analysis. For each of the tested foods, residual signal scores will be compared between migraine headache and controls using a permutation test on a two-sample t-test with a relative high number of resamplings (e.g., >1,000, or >10,000, or even >50,000). The Satterthwaite approximation can then be used for the denominator degrees of freedom to account for lack of homogeneity of variances, and the 2-tailed permuted p-value will represent the raw p-value for each food. False Discovery Rates (FDR) among the comparisons, will be adjusted by any acceptable statistical procedures (e.g., Benjamini-Hochberg, Family-wise Error Rate (FWER), Per Comparison Error Rate (PCER), etc.).

Foods were then ranked according to their 2-tailed FDR multiplicity-adjusted p-values. Foods with adjusted p-values equal to or lower than the desired FOR threshold are deemed to have significantly higher signal scores among migraine headaches than control subjects and therefore deemed candidates for inclusion into a food intolerance panel. A typical result that is representative of the outcome of the statistical procedure is provided in **Table 2.** Here, the ranking of foods is according to 2-tailed permutation T-test p-values with FDR adjustment.

Based on earlier experiments (data not shown here; see US 62/079783) the inventors contemplate that even for the same food preparation tested, the ELISA score for at least several food items will vary dramatically, and exemplary raw data are provided in **Table 3.** As should be readily appreciated, data unstratified by gender will therefore lose significant explanatory power where the same cutoff value is applied to raw data for male and female data. To overcome such disadvantage, the inventors therefore contemplate stratification of the data by gender as described below.

Statistical Method for Cutpoint Selection for each Food: The determination of what ELISA signal scores would constitute a "positive" response can be made by summarizing the distribution of signal scores among the Control subjects. For each food, migraine headache subjects who have observed scores greater than or equal to selected quantiles of the Control subject distribution will be deemed "positive". To attenuate the influence of any one subject on cutpoint determination, each food-specific and gender-specific dataset will be bootstrap resampled 1,000 times. Within each bootstrap replicate, the 90th and 95th percentiles of the Control signal scores will be determined. Each migraine headache subject in the bootstrap sample will be compared to the 90th and 95% percentiles to determine whether he/she had a "positive" response. The final 90th and 95th percentile-based cutpoints for each food and gender will be computed as the average 90th and 95th percentiles across the 1000 samples. The number of foods for which each migraine headache subject will be rated as "positive" was computed by pooling data across foods. Using such method, the inventors will be now able to identify cutoff values for a predetermined percentile rank that in most cases was substantially different as can be taken from **Table 4.**

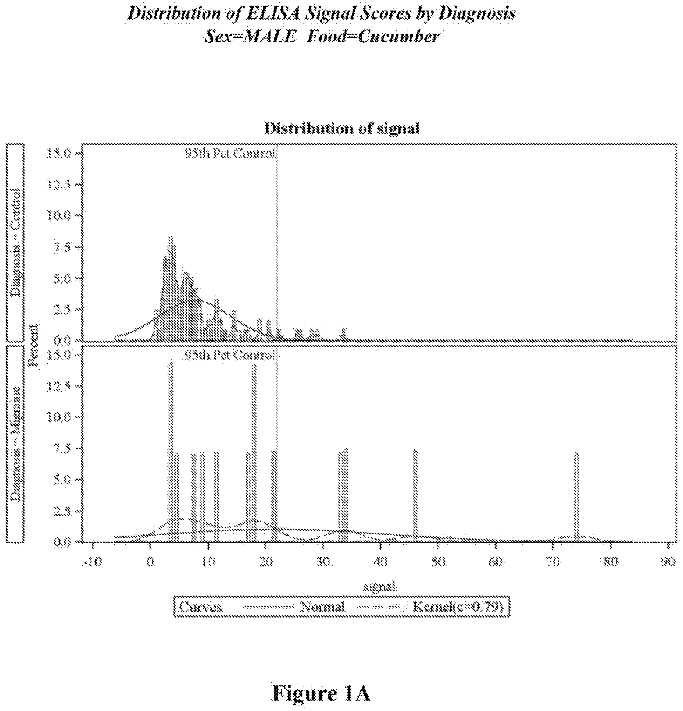

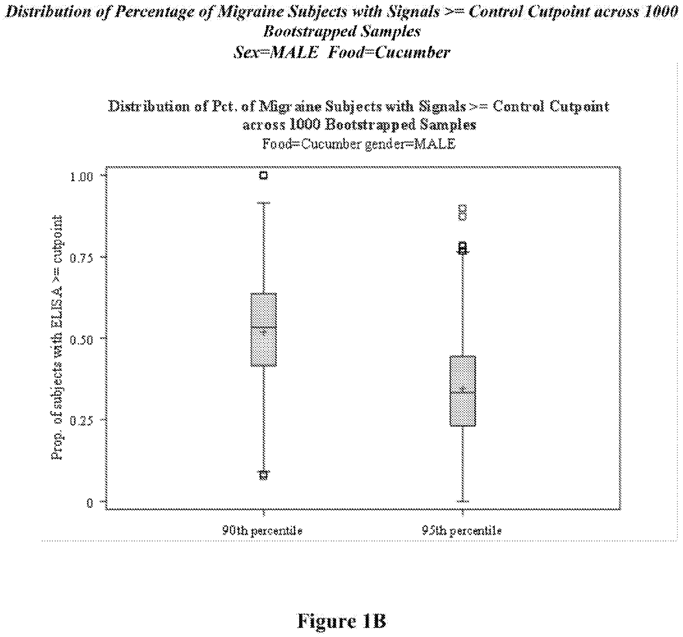

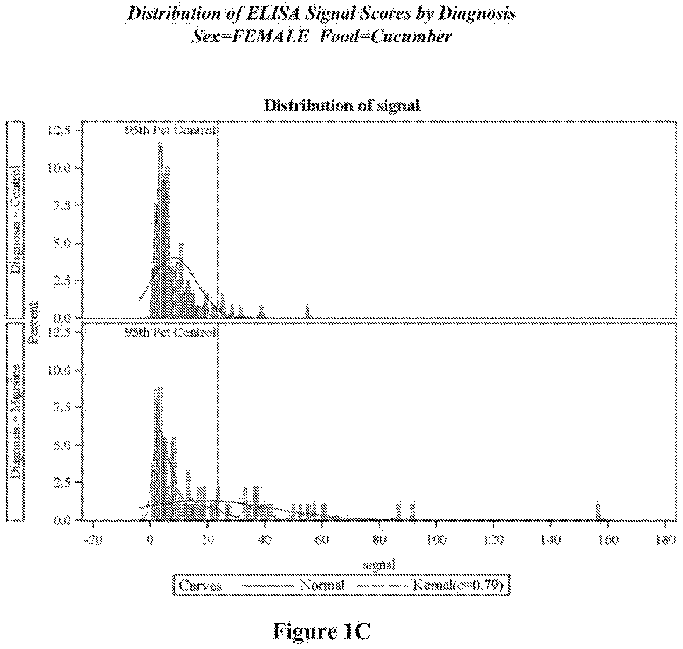

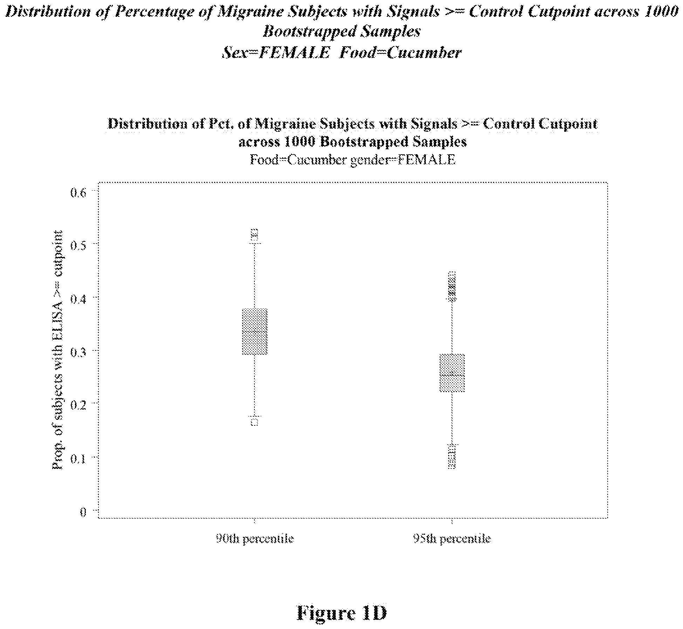

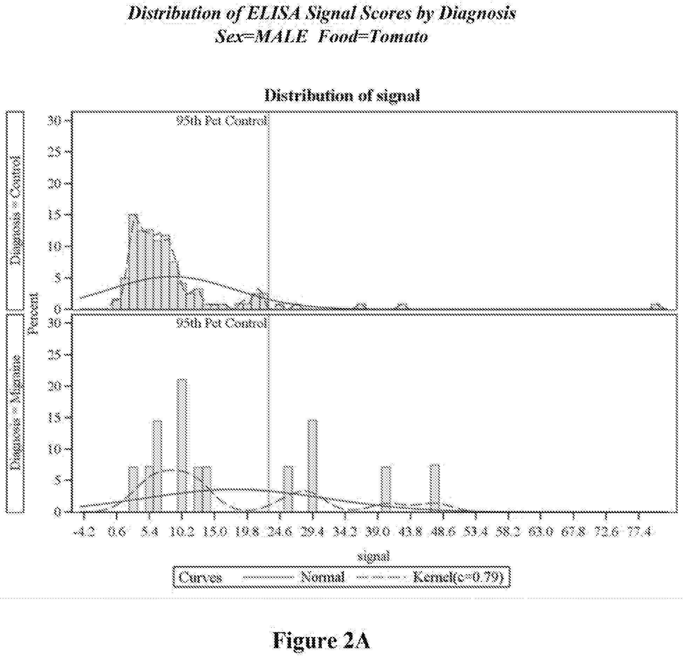

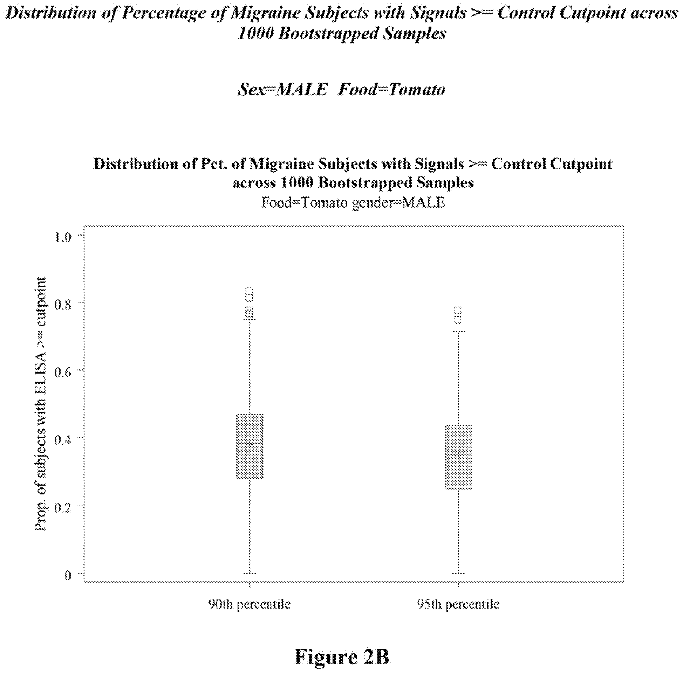

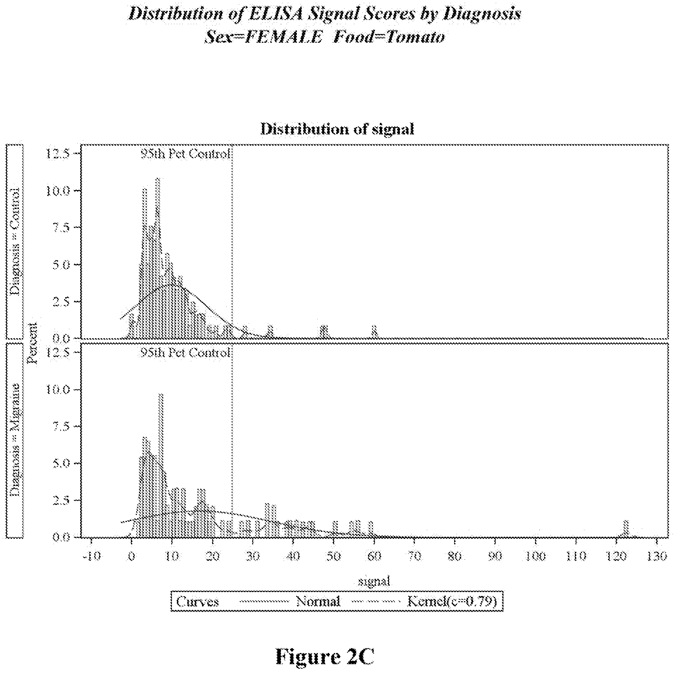

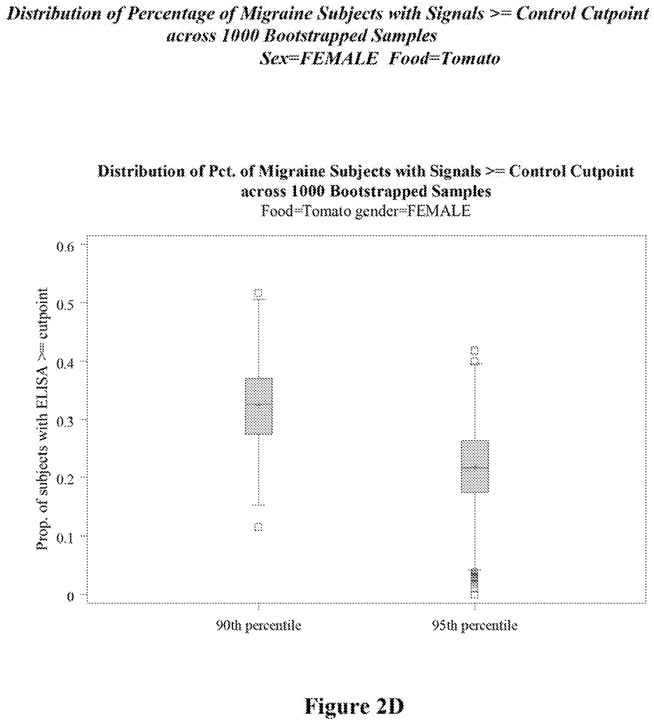

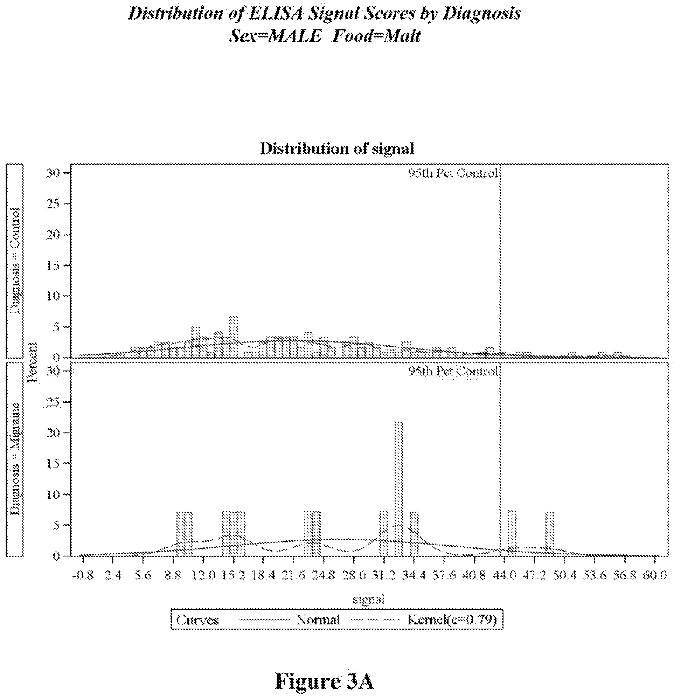

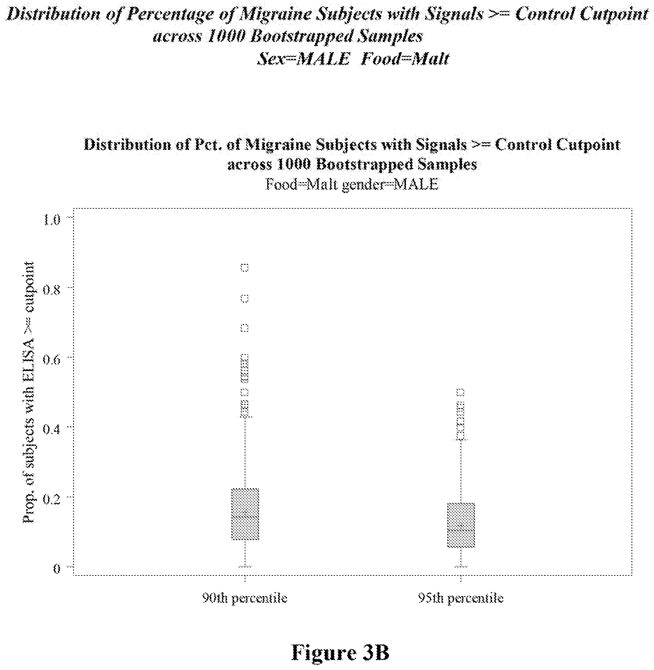

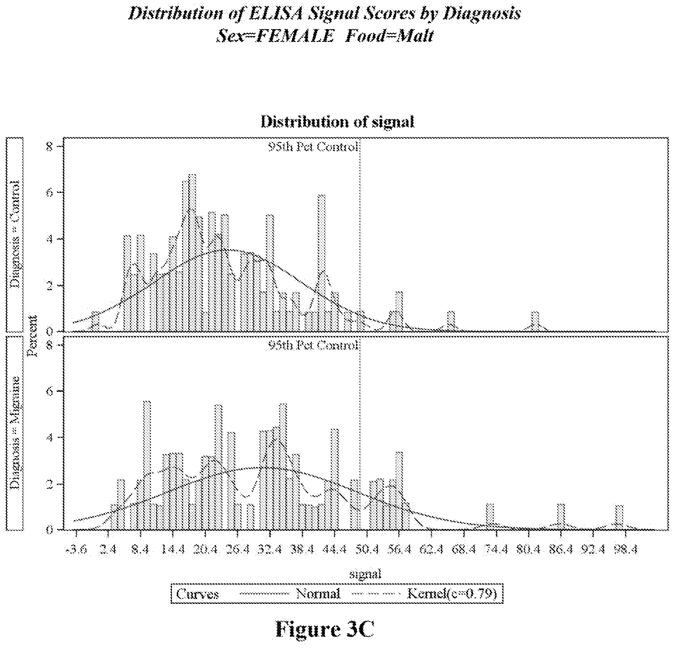

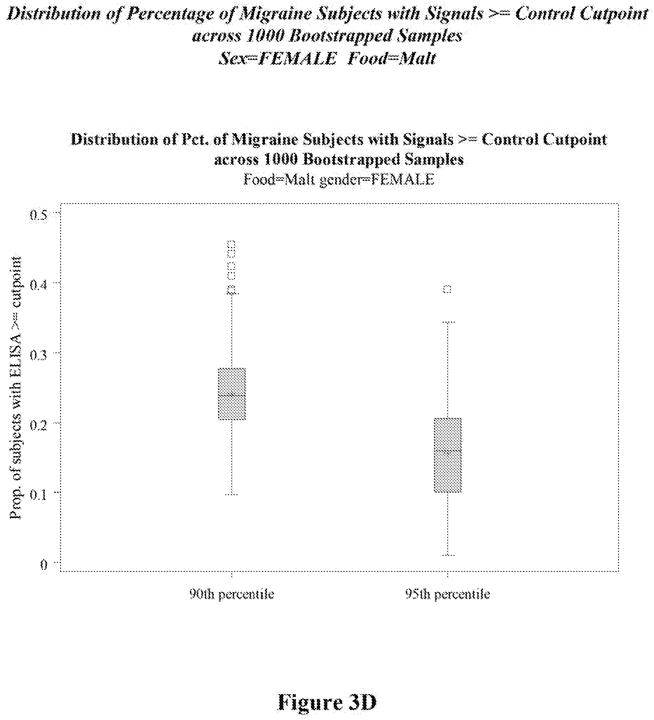

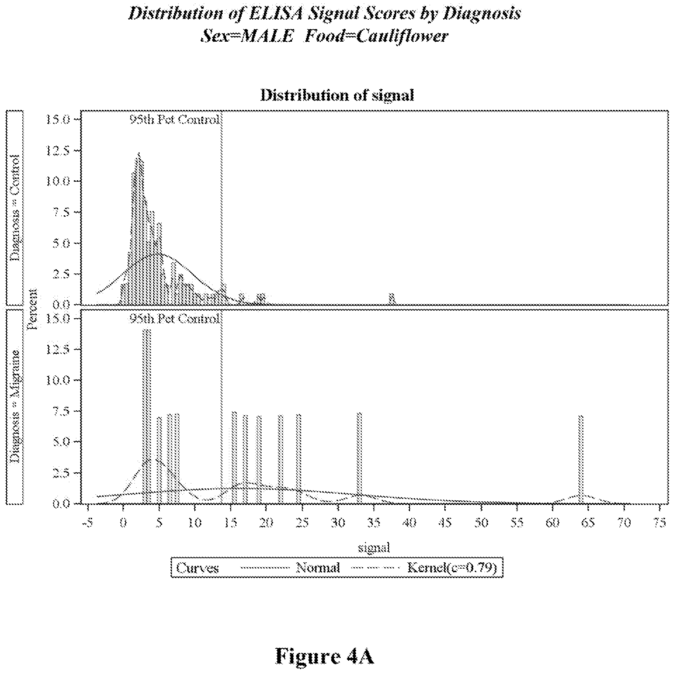

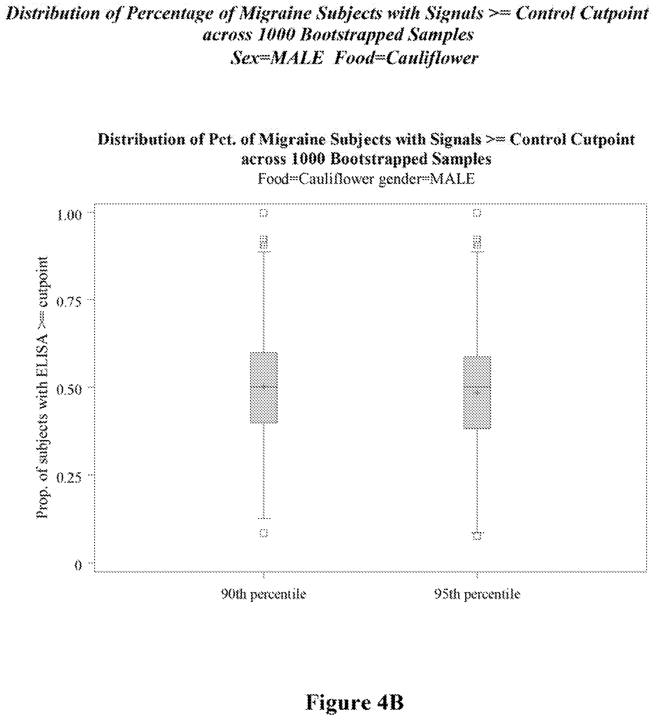

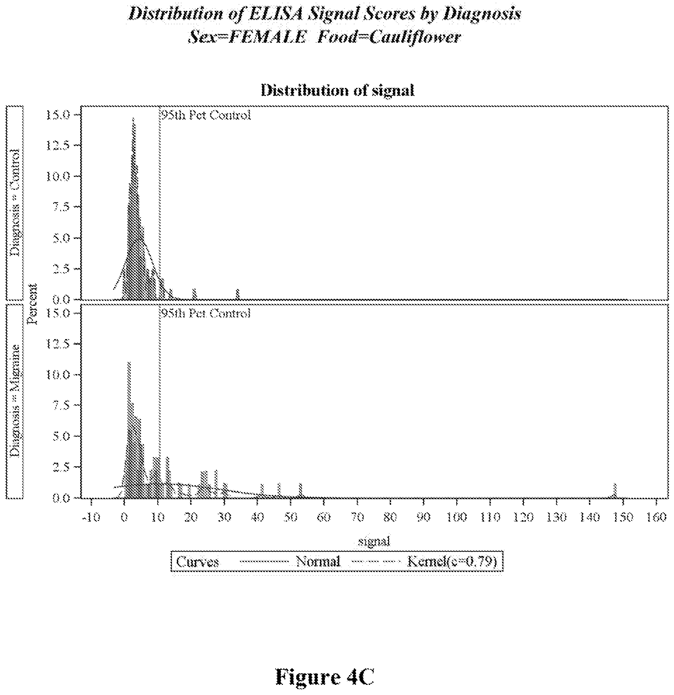

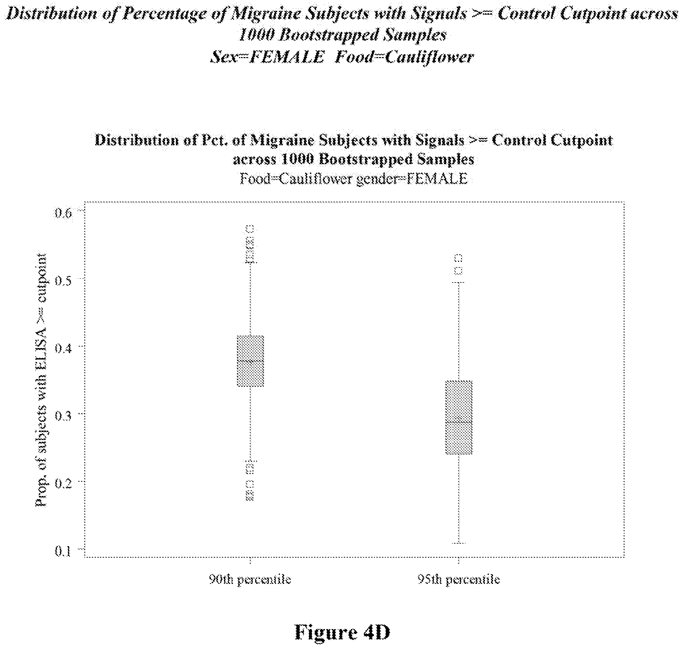

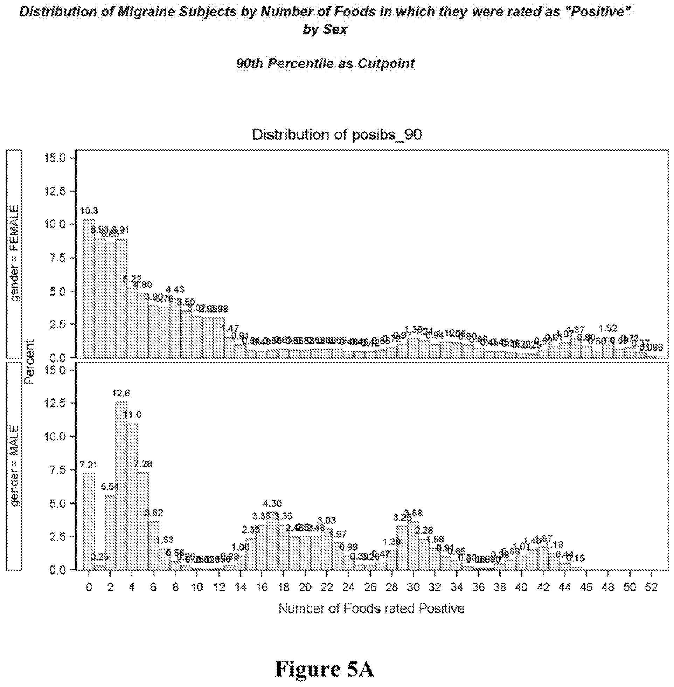

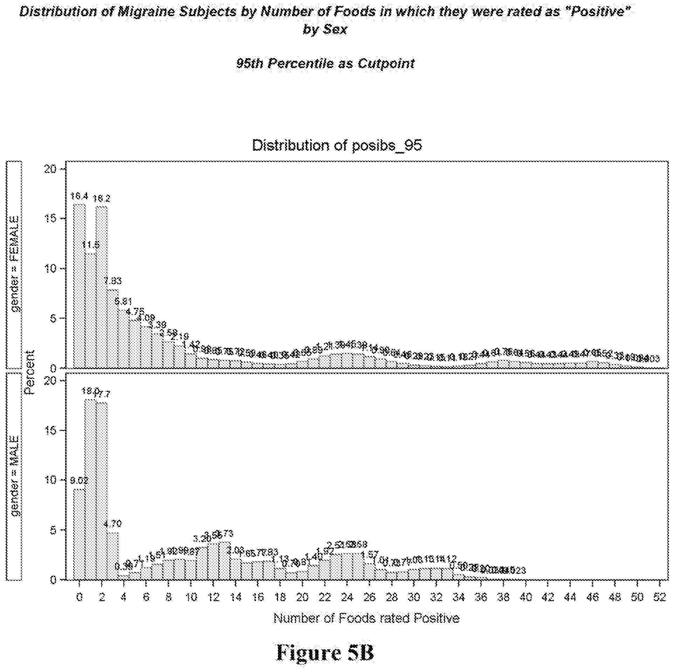

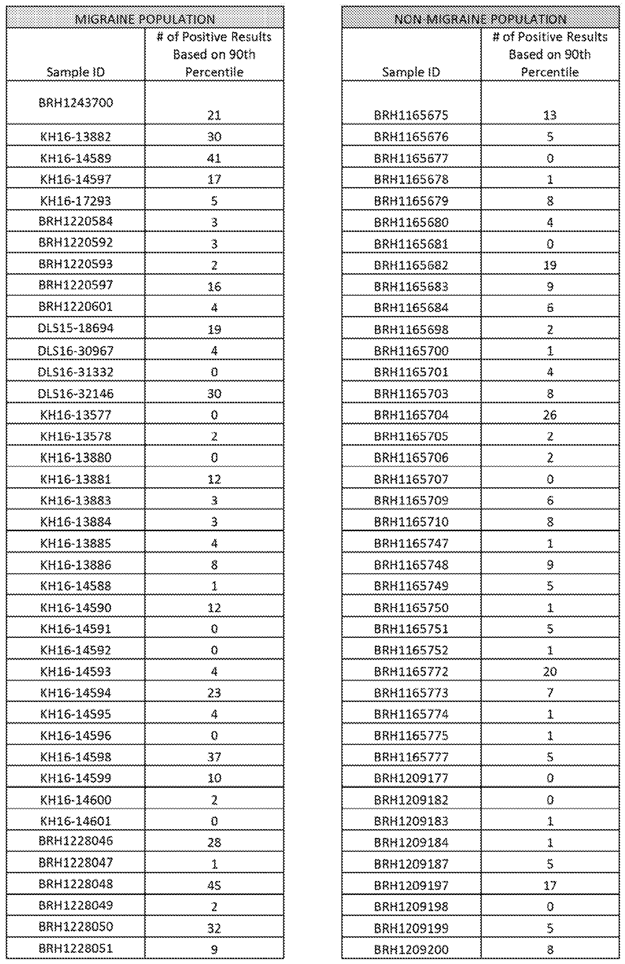

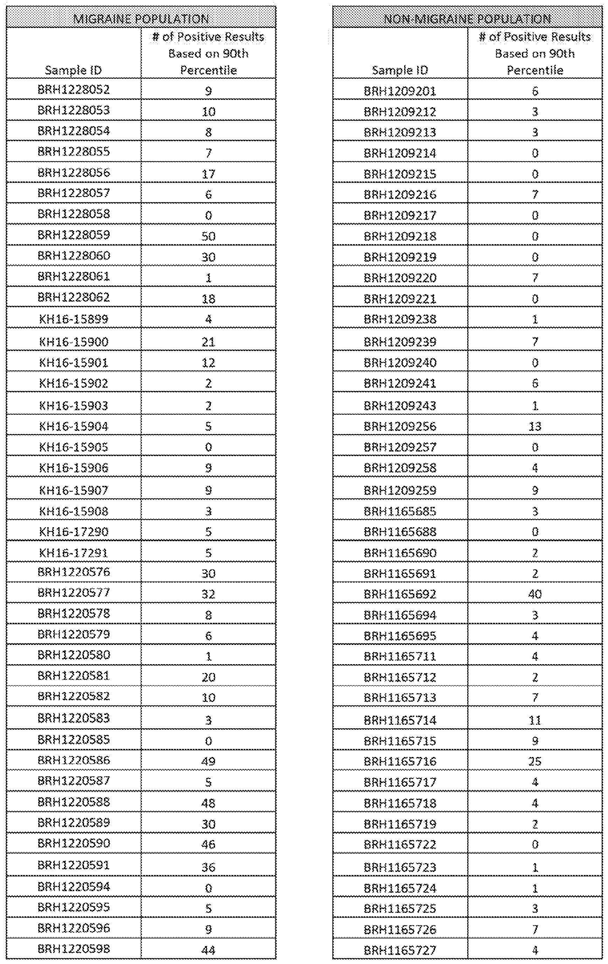

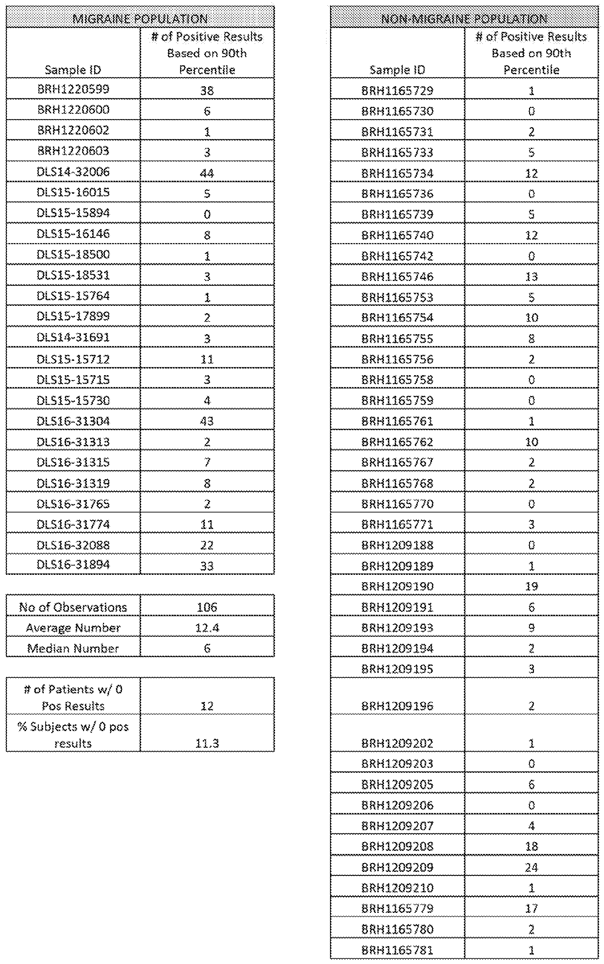

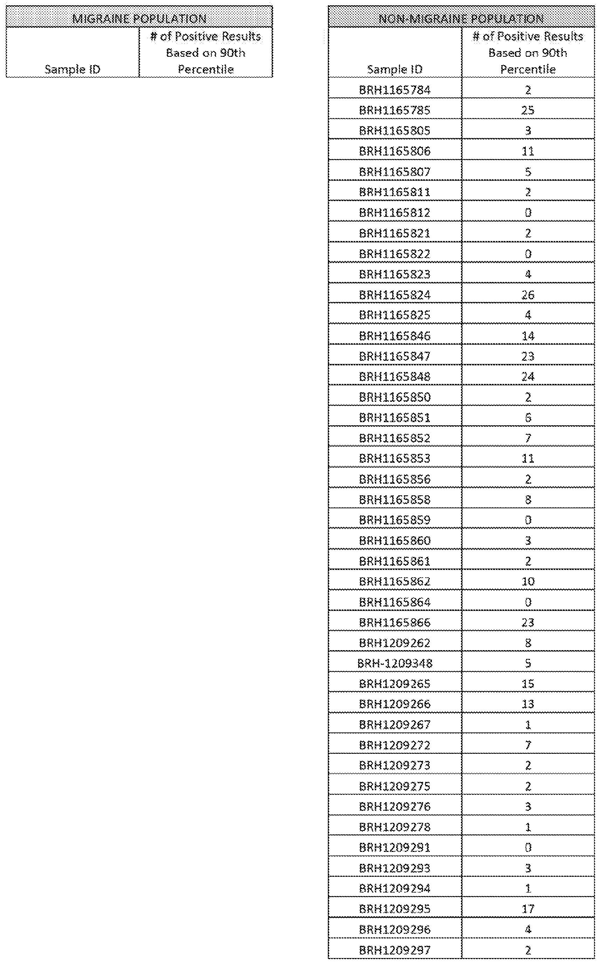

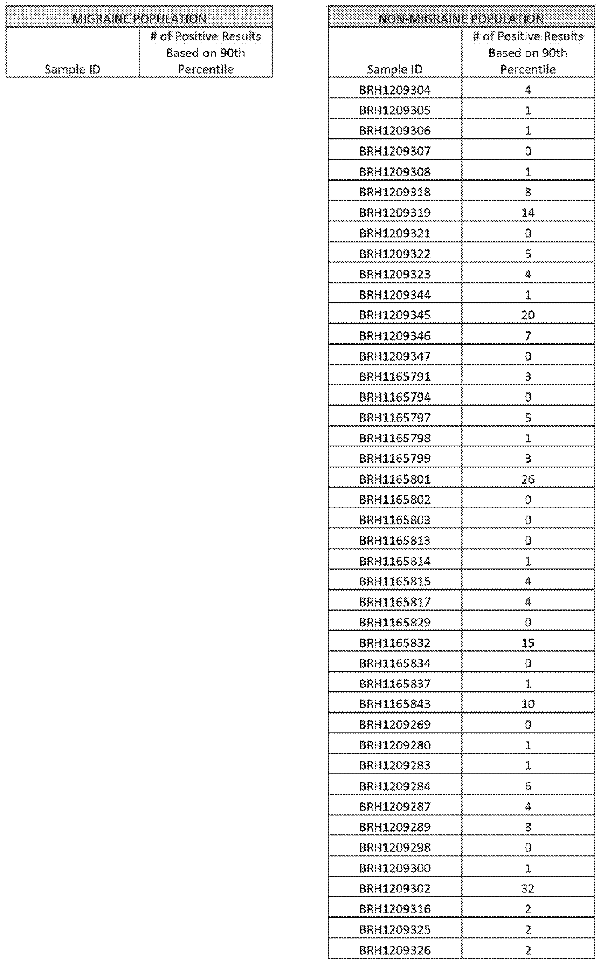

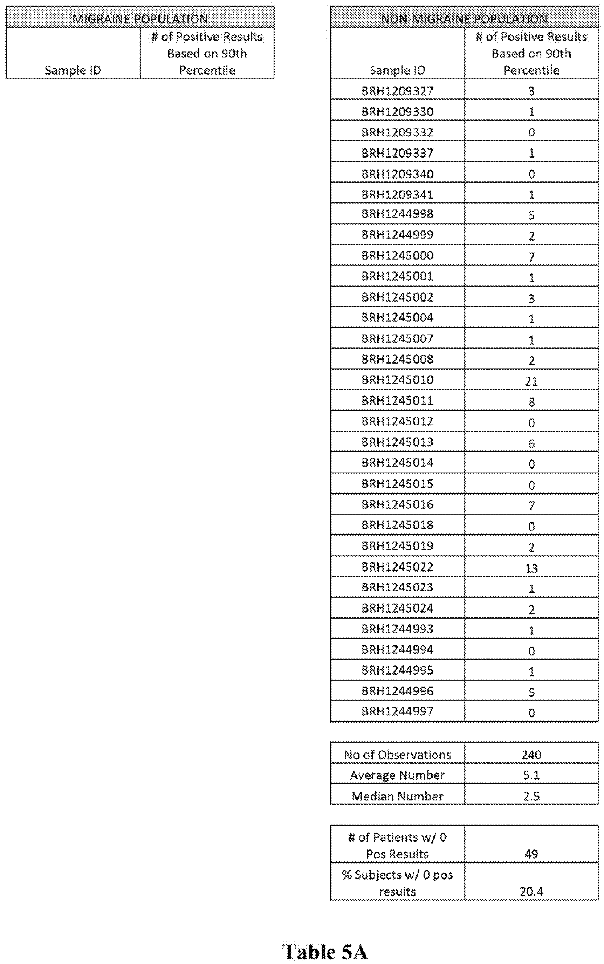

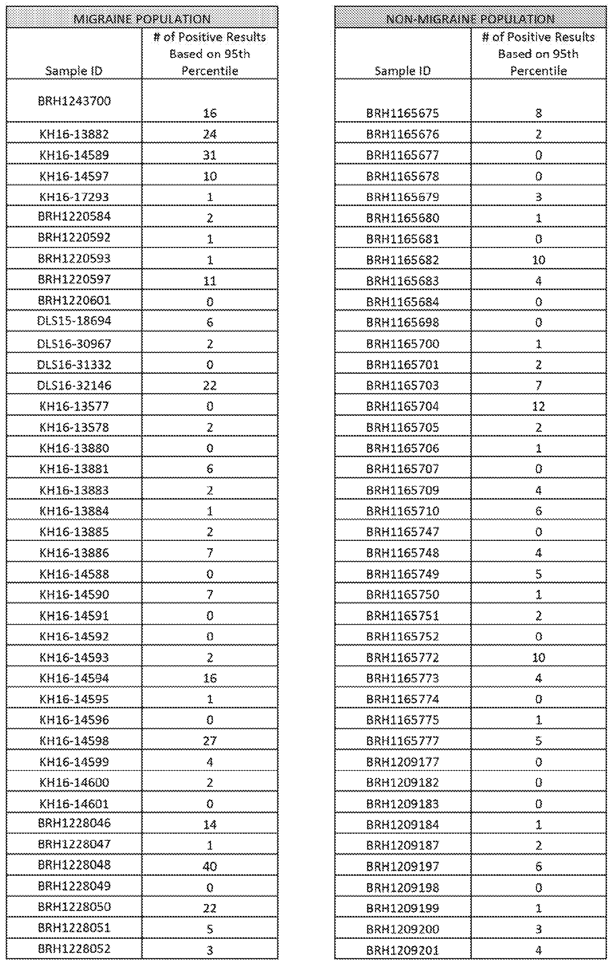

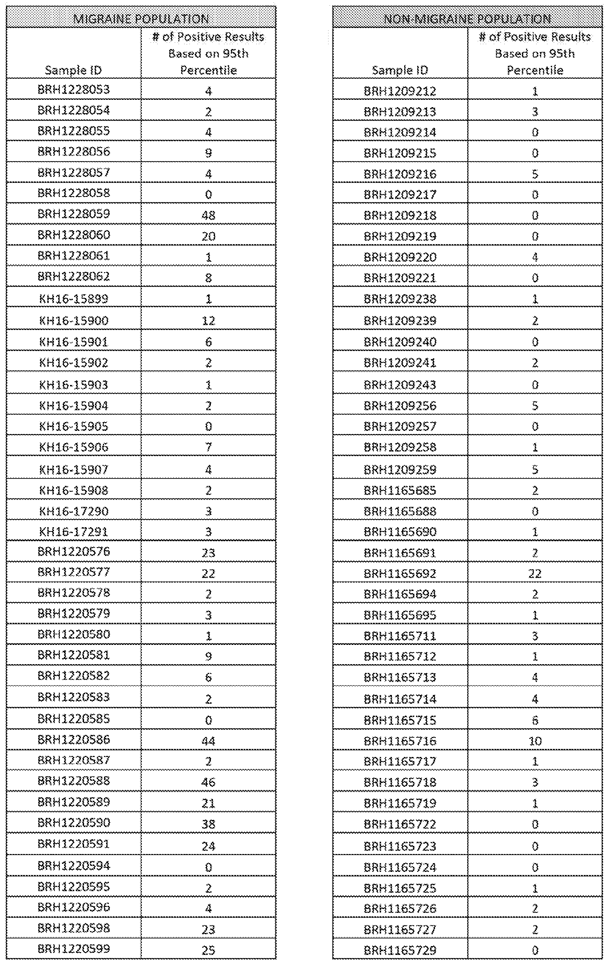

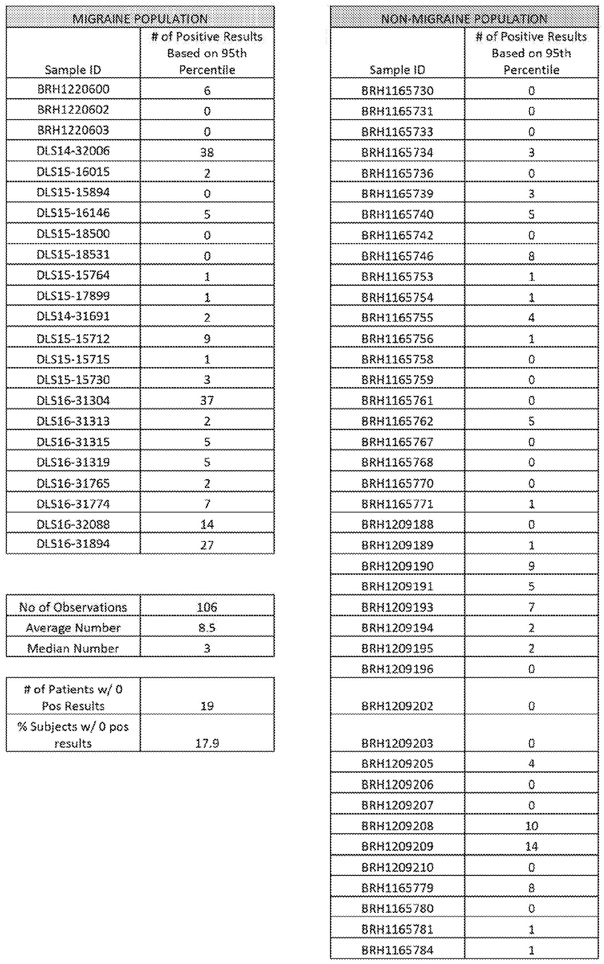

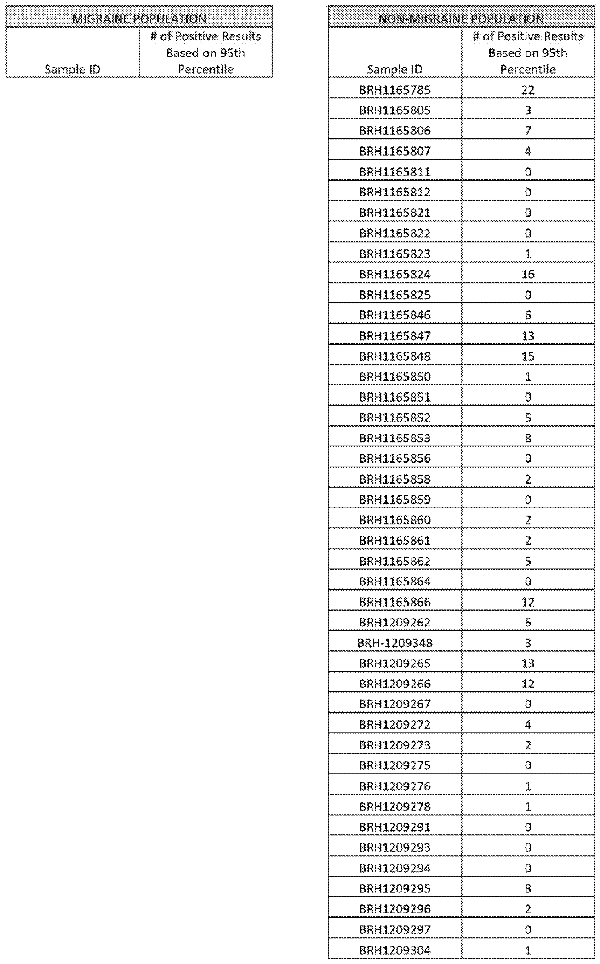

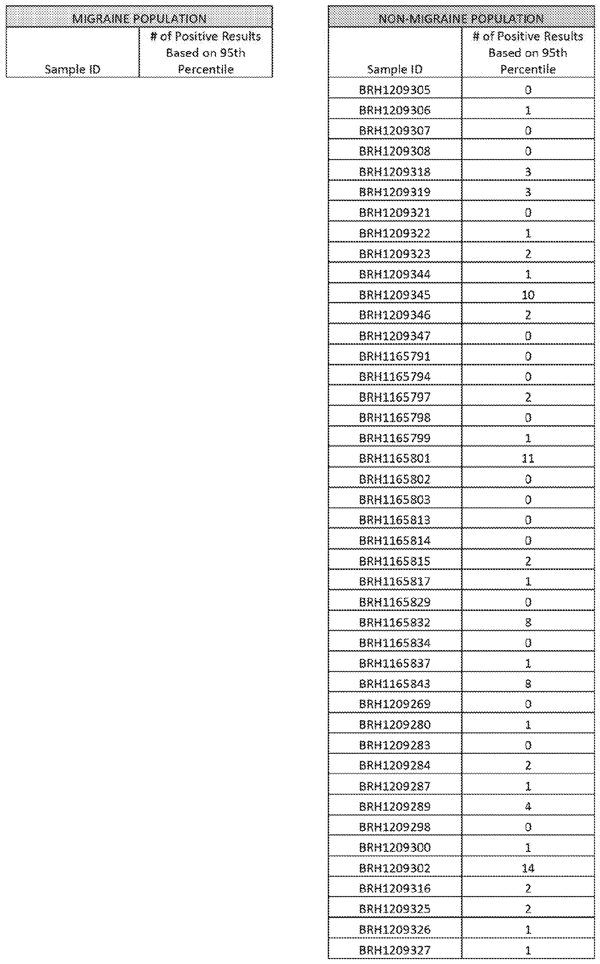

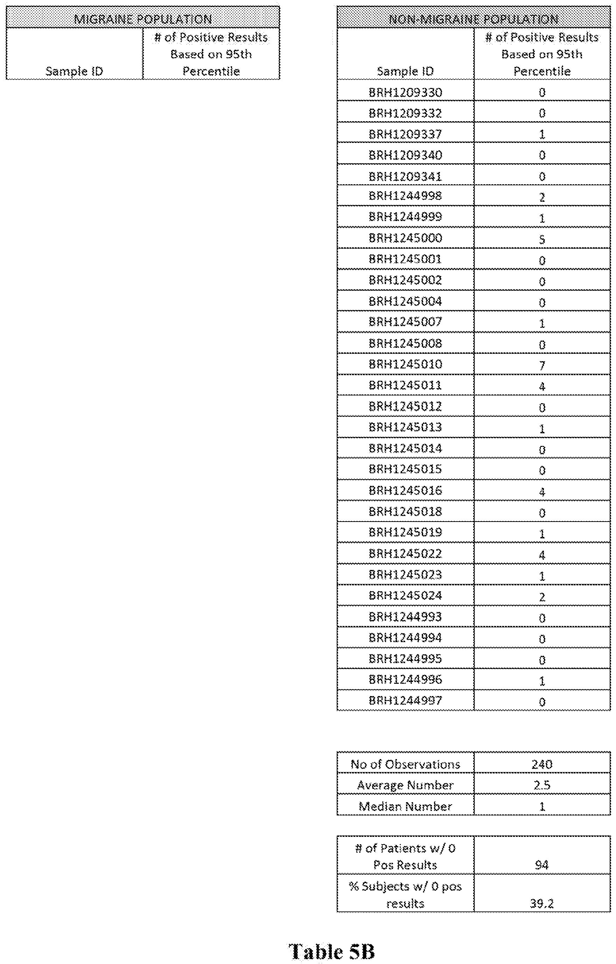

Typical examples for the gender difference in IgG response in blood with respect to cucumber is shown in **Figures 1A-1D****,** where **Figure 1A** shows the signal distribution in men along with the 95^{th} percentile cutoff as determined from the male control population. **Figure 1B** shows the distribution of percentage of male migraine headache subjects exceeding the 90^{th} and 95^{th} percentile, while **Figure 1C** shows the signal distribution in women along with the 95^{th} percentile cutoff as determined from the female control population. **Figure 1D** shows the distribution of percentage of female migraine headache subjects exceeding the 90^{th} and 95^{th} percentile. In the same fashion, **Figures 2A-2D** exemplarily depict the differential response to tomato, **Figures 3A-3D** exemplarily depict the differential response to malt, and **Figures 4A-4D** exemplarily depict the differential response to cauliflower. **Figures 5A-5B** show the distribution of migraine headache subjects by number of foods that were identified as trigger foods at the 90^{th} percentile (5A) and 95^{th} percentile (5B). Inventors contemplate that regardless of the particular food items, male and female responses were notably distinct.

It should be noted that nothing in the art has provided any predictable food groups related to migraine headaches that are gender-stratified. Thus, a discovery of food items that show distinct responses by gender is a surprising result, which was not expected by the inventors In other words, selection of food items based on gender stratification provides an unexpected technical effect such that statistical significances for particular food items as triggering foods among male or female migraine headache patients have been significantly improved.

Normalization of IgG Response Data: While the raw data of the patient's IgG response results can be use to compare strength of response among given foods, it is also contemplated that the IgG response results of a patient are normalized and indexed to generate unit-less numbers for comparison of relative strength of response to a given food. For example, one or more of a patient's food specific IgG results (*e.g*., IgG specific to cucumber and IgG specific to tomato) can be normalized to the patient's total IgG. The normalized value of the patient's IgG specific to cucumber can be 0.1 and the normalized value of the patient's IgG specific to tomato can be 0.3. In this scenario, the relative strength of the patient's response to tomato is three times higher compared to cucumber. Then, the patient's sensitivity to grapefruit and malt can be indexed as such.

In other examples, one or more of a patient's food specific IgG results (*e.g.,* IgG specific to shrimp and IgG specific to pork, etc.) can be normalized to the global mean of that patient's food specific IgG results. The global means of the patient's food specific IgG can be measured by total amount of the patient's food specific IgG. In this scenario, the patient's specific IgG to shrimp can be normalized to the mean of patient's total food specific IgG (*e.g..* mean of IgG levels to shrimp, pork, Dungeness crab, chicken, peas, etc.). However, it is also contemplated that the global means of the patient's food specific IgG can be measured by the patient's IgG levels to a specific type of food via multiple tests. If the patient has been tested for his sensitivity to shrimp five times and to pork seven times previously, the patient's new IgG values to shrimp or to pork are normalized to the mean of five-times test results to shrimp or the mean of seven-times test results to pork. The normalized value of the patient's IgG specific to shrimp can be 6.0 and the normalized value of the patient's IgG specific to pork can be 1.0. In this scenario, the patient has six times higher sensitivity to shrimp at this time compared to his average sensitivity to shrimp, but substantially similar sensitivity to pork. Then, the patient's sensitivity to shrimp and pork can be indexed based on such comparison.

Methodology to determine the subset of migraine headache patients with food sensitivities that underlie migraine headaches: While it is suspected that food sensitivities may play a substantial role in signs and symptoms of migraine headaches, some migraine headache patients may not have food sensitivities that underlie migraine headaches. Those patients may not be benefit from dietary intervention to treat signs and symptoms of migraine headaches. To determine the subset of such patients, body fluid samples of migraine headache patients and non- migraine headache patients can be tested with ELISA test using test devices with at least 6, or at least 12, or at least 24, or at least 48 food samples.

**Table 5A** and **Table 5B** provide exemplary raw data. As should be readily appreciated, the data indicate number of positive results out of 90 sample foods based on 90^{th} percentile value (Table 5A) or 95^{th} percentile value (Table 5B). The first column is migraine headache (n=106); second column is non-migraine headache (n=240) by ICD-10 code. Average and median number of positive foods was computed for migraine headache and non-migraine headache patients. From the raw data shown in Table 5A and Table 5B, average and standard deviation of the number of positive foods was computed for migraine headache and non-migraine headache patients. Additionally, the number and percentage of patients with zero positive foods was calculated for both migraine headache and non-migraine headache. The number and percentage of patients with zero positive foods in the migraine population is almost half of the percentage of patients with zero positive foods in the non-migraine population (1 1.3% vs. 20.4%, respectively) based on 90^{th} percentile value (Table 5A), and the percentage of patients in the migraine population with zero positive foods is also less than half of that seen in the non-migraine headache population (17.9% vs. 39.2%, respectively) based on 95^{th} percentile value (Table 5B). Thus, it can be easily appreciated that the migraine headache patient having sensitivity to zero positive foods is unlikely to have food sensitivities underlying their signs and symptoms of migraine headache.

**Table 6A** and **Table 7A** show exemplary statistical data summarizing the raw data of two patient populations shown in Table 5A. The statistical data includes normality, arithmetic mean, median, percentiles and 95% confidence interval (CI) for the mean and median representing number of positive foods in the migraine headache population and the non-migraine headache population. **Table 6B** and **Table 7B** show exemplary statistical data summarizing the raw data of two patient populations shown in Table 5B. The statistical data includes normality, arithmetic mean, median, percentiles and 95% confidence interval (CI) for the mean and median representing number of positive foods in the migraine headache population and the non-migraine headache population.

**Table 8A** and **Table 9A** show exemplary statistical data summarizing the raw data of two patient populations shown in Table 5A. In Tables 8A and 9A, the raw data was transformed by logarithmic transformation to improve the data interpretation. **Table 8B** and **Table 9B** show another exemplary statistical data summarizing the raw data of two patient populations shown in Table 5B. In Tables 8B and 9B, the raw data was transformed by logarithmic transformation to improve the data interpretation.

**Table 10A** and **Table 11A** show exemplary statistical data of an independent T-test (Table 10A, logarithmically transformed data) and a Mann-Whitney test (Table 11A) to compare the geometric mean number of positive foods between the migraine headache and non- migraine headache samples. The data shown in Table 10A and Table 11A indicate statistically significant differences in the geometric mean of positive number of foods between the migraine headache population and the non-migraine headache population. In both statistical tests, it is shown that the number of positive responses with 90 food samples is significantly higher in the migraine headache population than in the non-migraine headache population with an average discriminatory p-value of ≤ 0.0001. These statistical data is also illustrated as a box and whisker plot in **Figure 6A****,** and a notched box and whisker plot in **Figure 6B****.**

**Table 10B** and **Table 11B** show exemplary statistical data of an independent T-test (Table 10A, logarithmically transformed data) and a Mann-Whitney test (Table 11B) to compare the geometric mean number of positive foods between the migraine headache and non-migraine headache samples. The data shown in Table 10B and Table 11B indicate statistically significant differences in the geometric mean of positive number of foods between the migraine headache population and the non-migraine headache population. In both statistical tests, it is shown that the number of positive responses with 90 food samples is significantly higher in the migraine headache population than in the non-migraine headache population with an average discriminatory p-value of ≤ 0.0001. These statistical data is also illustrated as a box and whisker plot in **Figure 6C****,** and a notched box and whisker plot in **Figure 6D****.**

**Table 12A** shows exemplary statistical data of a Receiver Operating Characteristic (ROC) curve analysis of data shown in Tables 5A-11A to determine the diagnostic power of the test used in Table 5 at discriminating migraine headache from non-migraine headache subjects. When a cutoff criterion of more than 7 positive foods is used, the test yields a data with 46.2% sensitivity and 77.92% specificity, with an area under the curve (AUROC) of 0.664. The p-value for the ROC is significant at a p-value of <0.0001. **Figure 7A** illustrates the ROC curve corresponding to the statistical data shown in Table 12A. Because the statistical difference between the migraine headache population and the non-migraine headache population is significant when the test results are cut off to a positive number of 7, the number of foods for which a patient tests positive could be used as a confirmation of the primary clinical diagnosis of migraine headaches, and whether it is likely that food sensitivities underlies on the patient's signs and symptoms of migraine headache. Therefore, the above test can be used as another 'rule in' test to add to currently available clinical criteria for diagnosis for migraine headache.

As shown in Tables 5A-12A, and Figure 7A, based on 90^{th} percentile data, the number of positive foods seen in migraine headache vs. non-migraine headache subjects is significantly different whether the geometric mean or median of the data is compared. The number of positive foods that a person has is indicative of the presence of migraine headaches in subjects. The test has discriminatory power to detect migraine headache with ~46% sensitivity and ~78% specificity. Additionally, the absolute number and percentage of subjects with 0 positive foods is also very different in migraine headache vs. non-migraine headache subjects, with a far lower percentage of migraine headache subjects (11%) having 0 positive foods than non-migraine headache subjects (20%). The data suggests a subset of migraine headache patients may have migraine headaches due to other factors than diet, and may not benefit from dietary restriction.

**Table 12B** shows exemplary statistical data of a Receiver Operating Characteristic (ROC) curve analysis of data shown in Tables 5B-11B to determine the diagnostic power of the test used in Table 5 at discriminating migraine headache from non-migraine headache subjects. When a cutoff criterion of more than 1 positive foods is used, the test yields a data with 69.8% sensitivity and 58.3% specificity, with an area under the curve (AUROC) of 0..681. The p-value for the ROC is significant at a p-value of <0.0001. **Figure 7B** illustrates the ROC curve corresponding to the statistical data shown in Table 12B. Because the statistical difference between the migraine headache population and the non-migraine headache population is significant when the test results are cut off to positive number of 1, the number of foods that a patient tests positive could be used as a confirmation of the primary clinical diagnosis of migraine headache, and whether it is likely that food sensitivities underlies on the patient's signs and symptoms of migraine headache. Therefore, the above test can be used as another 'rule in' test to add to currently available clinical criteria for diagnosis for migraine headaches.

As shown in Tables 5B-12B, and Figure 7B, based on 95^{th} percentile data, the number of positive foods seen in migraine headache vs. non-migraine headache subjects is significantly different whether the geometric mean or median of the data is compared. The number of positive foods that a person has is indicative of the presence of migraine headaches in subjects. The test has discriminatory power to detect migraine headaches with ~70% sensitivity and ~60% specificity. Additionally, the absolute number and percentage of subjects with 0 positive foods is also very different in migraine headache vs. non-migraine headache subjects, with a far lower percentage of migraine headache subjects (18%) having 0 positive foods than non- migraine headache subjects (39%). The data suggests a subset of migraine headache patients may have migraine headache due to other factors than diet, and may not benefit from dietary restriction.

Method for determining distribution of per-person number of foods declared "positive": To determine the distribution of number of "positive" foods per person and measure the diagnostic performance, the analysis was performed with 90 food items from the Table 1, which shows most positive responses to migraine headache patients. The 90 food items includes chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, halibut, cabbage, orange, rice, safflower, tomato, almond, oat, barley, peach, grape, potato, spinach, sole, and butter. To attenuate the influence of any one subject on this analysis, each food-specific and gender-specific dataset was bootstrap resampled 1000 times. Then, for each food item in the bootstrap sample, sex-specific cutpoint was determined using the 90th and 95th percentiles of the control population. Once the sex-specific cutpoints were determined, the sex-specific cutpoints was compared with the observed ELISA signal scores for both control and migraine headache subjects. In this comparison, if the observed signal is equal or more than the cutpoint value, then it is determined "positive" food, and if the observed signal is less than the cutpoint value, then it is determined "negative" food.

Once all food items were determined either positive or negative, the results of the 180 (90 foods x 2 cutpoints) calls for each subject were saved within each bootstrap replicate. Then, for each subject, 90 calls were summed using 90^{th} percentile as cutpoint to get "Number of Positive Foods (90^{th})," and the rest of 90 calls were summed using 95^{th} percentile to get "Number of Positive Foods (95^{th})." Then, within each replicate, "Number of Positive Foods (90^{th})" and "Number of Positive Foods (95^{th})" were summarized across subjects to get descriptive statistics for each replicate as follows: 1) overall means equals to the mean of means, 2) overall standard deviation equals to the mean of standard deviations, 3) overall medial equals to the mean of medians, 4) overall minimum equals to the minimum of minimums, and 5) overall maximum equals to maximum of maximum. In this analysis, to avoid non-integer "Number of Positive Foods" when computing frequency distribution and histogram, the authors pretended that the 1000 repetitions of the same original dataset were actually 999 sets of new subjects of the same size added to the original sample. Once the summarization of data is done, frequency distributions and histograms were generated for both "Number of Positive Foods (90^{th})" and "Number of Positive Foods (95^{th})" for both genders and for both migraine headache subjects and control subjects using programs "a_pos_foods.sas, a_pos_foods_by_dx.sas".

Method for measuring diagnostic performance: To measure diagnostic performance for each food items for each subject, we used data of "Number of Positive Foods (90^{th})" and "Number of Positive Foods (95^{th})" for each subject within each bootstrap replicate described above. In this analysis, the cutpoint was set to 1. Thus, if a subject has one or more "Number of Positive Foods (90^{th})", then the subject is called "Has migraine headache." If a subject has less than one "Number of Positive Foods (90^{th})", then the subject is called "Does Not Have migraine headache." When all calls were made, the calls were compared with actual diagnosis to determine whether a call was a True Positive (TP), True Negative (TN), False Positive (FP), or False Negative (FN). The comparisons were summarized across subjects to get the performance metrics of sensitivity, specificity, positive predictive value, and negative predictive value for both "Number of Positive Foods(90^{th})" and "Number of Positive Foods(95^{th})" when the cutpoint is set to 1 for each method. Each (sensitivity, 1-specificity) pair becomes a point on the ROC curve for this replicate.

To increase the accuracy, the analysis above was repeated by incrementing cutpoint from 2 up to 24, and repeated for each of the 1000 bootstrap replicates. Then the performance metrics across the 1000 bootstrap replicates were summarized by calculating averages using a program "t_pos_foods_by_dx.sas". The results of diagnostic performance for female and male are shown in Table 13 (90^{th} percentile) and Table 14 (95^{th} percentile).

## Claims

1. A test panel for testing for food intolerance in a patient diagnosed with, or suspected of having, migraine headaches, the test panel comprising:
a plurality of distinct food preparations, wherein each food preparation is
independently coupled to an individually addressable solid carrier;
wherein the plurality of distinct food preparations consists of cucumber, tomato, malt, cauliflower, broccoli, peach, cantaloupe, orange, egg, tea, cabbage, green pepper, safflower, grapefruit, Swiss cheese, chocolate, wheat, cow's milk, rye, baker's yeast, cottage cheese, brewer's yeast, oat, honey, almond, sweet potato, onion, lemon, cheddar cheese, butter, rice, cane sugar, parsley, mustard, tobacco, goat's milk, American cheese, yogurt, eggplant, black walnut, spinach, cola nut, avocado, corn, garlic, pineapple, strawberry, sunflower seed, buck wheat, beef, potato, and mushroom.

2. The test panel of claim 1, wherein each of the distinct food preparations comprises a crude filtered aqueous extract or a processed aqueous extract.

3. The test panel of claim 1 or claim 2, wherein the solid carrier is a well of a multiwell plate, a bead, an electrical sensor, a chemical sensor, a microchip or an adsorptive film.

4. An *in vitro* method of testing food intolerance in patients that are diagnosed with or suspected to have migraine headaches, comprising:
contacting a food preparation, said food preparation having at least one component, with a bodily fluid of a patient that is diagnosed with or suspected of having migraine headaches, wherein the bodily fluid comprises at least one immunoglobulin, wherein the bodily fluid is associated with a gender identification, and wherein the contacting is performed under conditions that allow at least a portion of the immunoglobulin to bind to the at least one component;
measuring the portion of the immunoglobulin bound to the at least one component of the food preparation to obtain a signal;
comparing the signal to a gender-stratified reference value for the food preparation using the gender identification to obtain a result; and
updating or generating a report using the result,
wherein the contacting a food preparation is performed with a multiplexed assay comprising a plurality of distinct food preparations; and
wherein the plurality of distinct food preparations consists of cucumber, tomato, malt, cauliflower, broccoli, peach, cantaloupe, orange, egg, tea, cabbage, green pepper, safflower, grapefruit, Swiss cheese, chocolate, wheat, cow's milk, rye, baker's yeast, cottage cheese, brewer's yeast, oat, honey, almond, sweet potato, onion, lemon, cheddar cheese, butter, rice, cane sugar, parsley, mustard, tobacco, goat's milk, American cheese, yogurt, eggplant, black walnut, spinach, cola nut, avocado, corn, garlic, pineapple, strawberry, sunflower seed, buck wheat, beef, potato, and mushroom.

5. The method of claim 4, wherein the bodily fluid of the patient comprises whole blood, plasma, serum, saliva, or a fecal suspension.

6. The method of claim 4 or claim 5, wherein the gender-stratified reference value for each of the food preparations is the 90^{th} percentile rank, or higher, of signals obtained by contacting bodily fluid from a control group of subjects that is not diagnosed with or suspected of having migraine headaches with the food preparation.

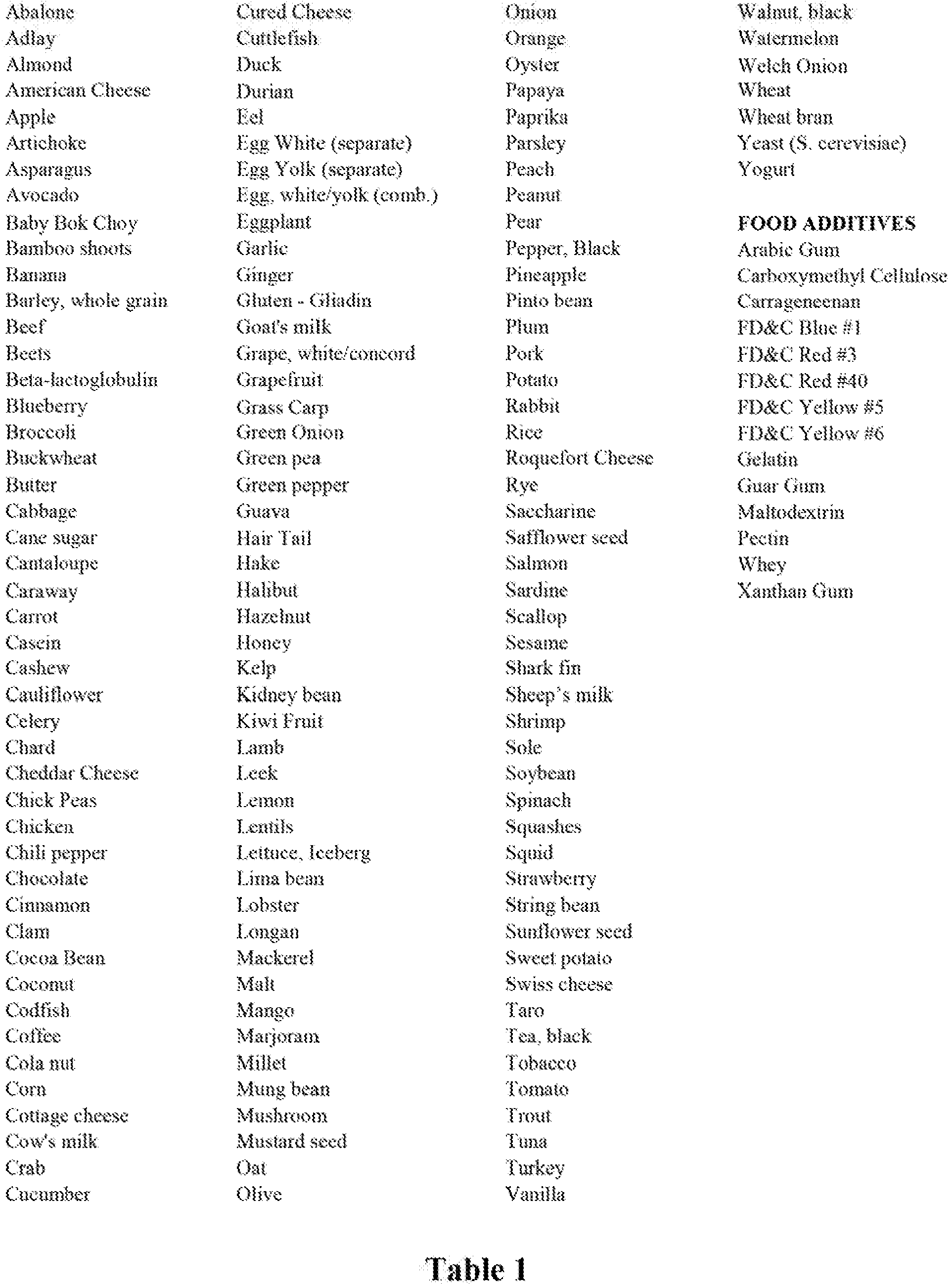

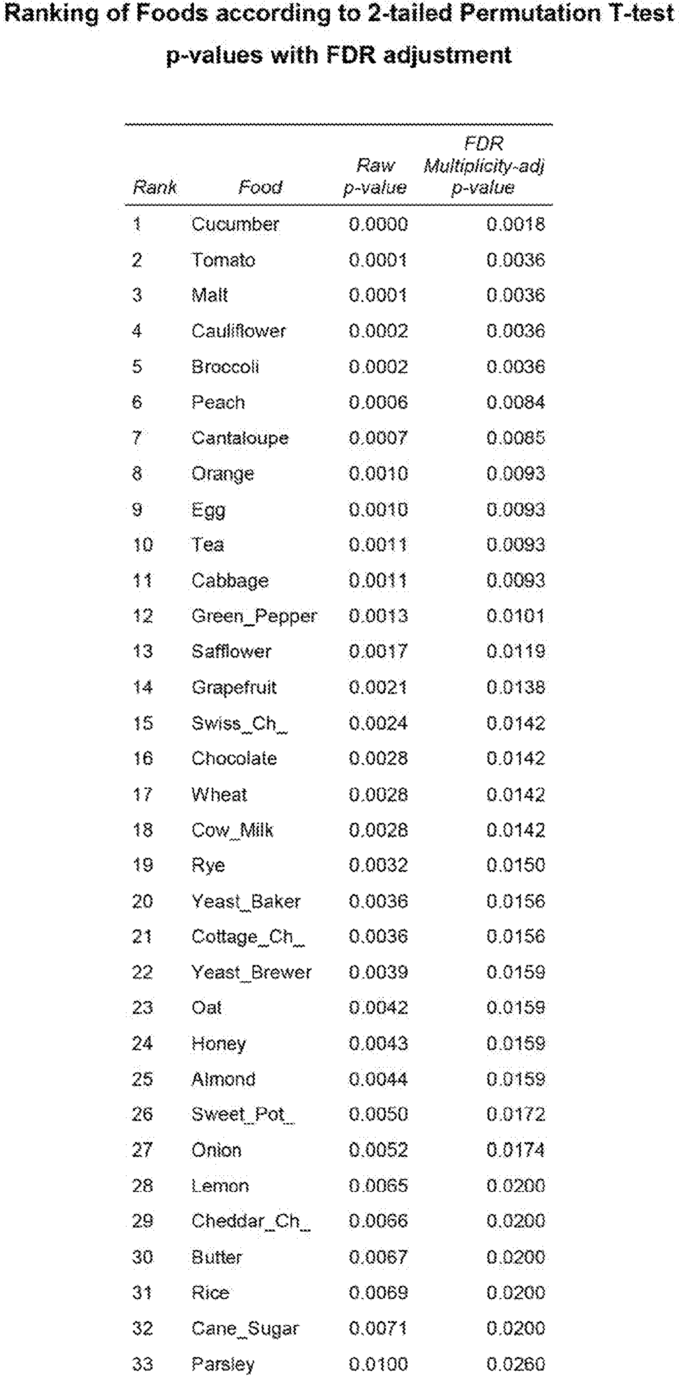

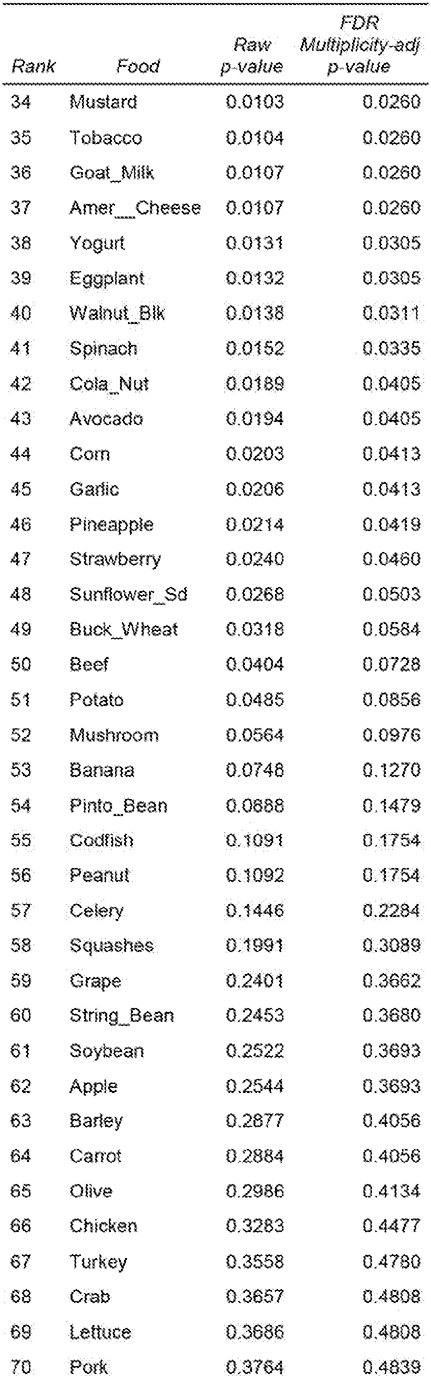

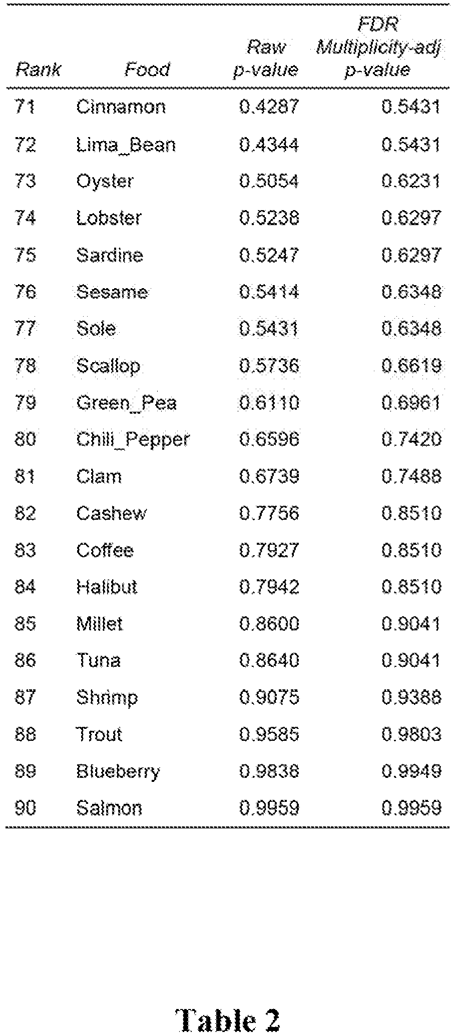

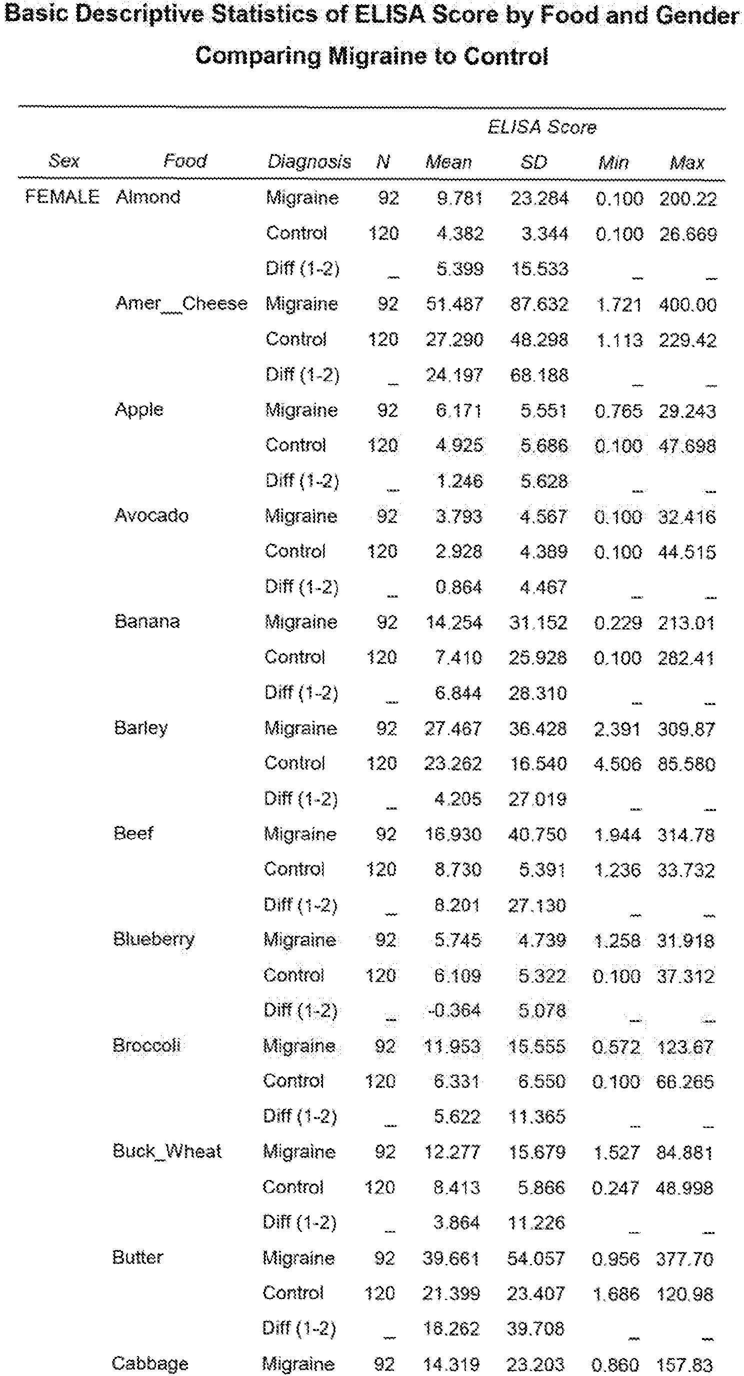

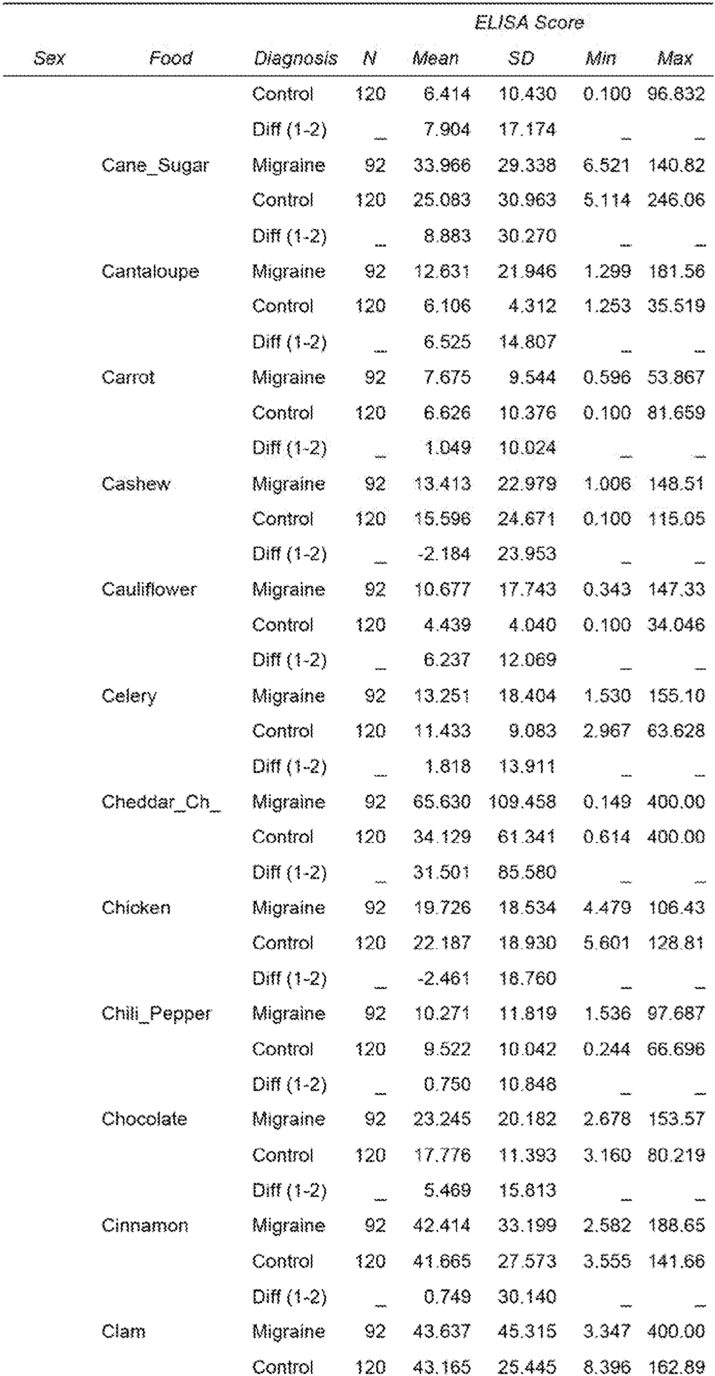

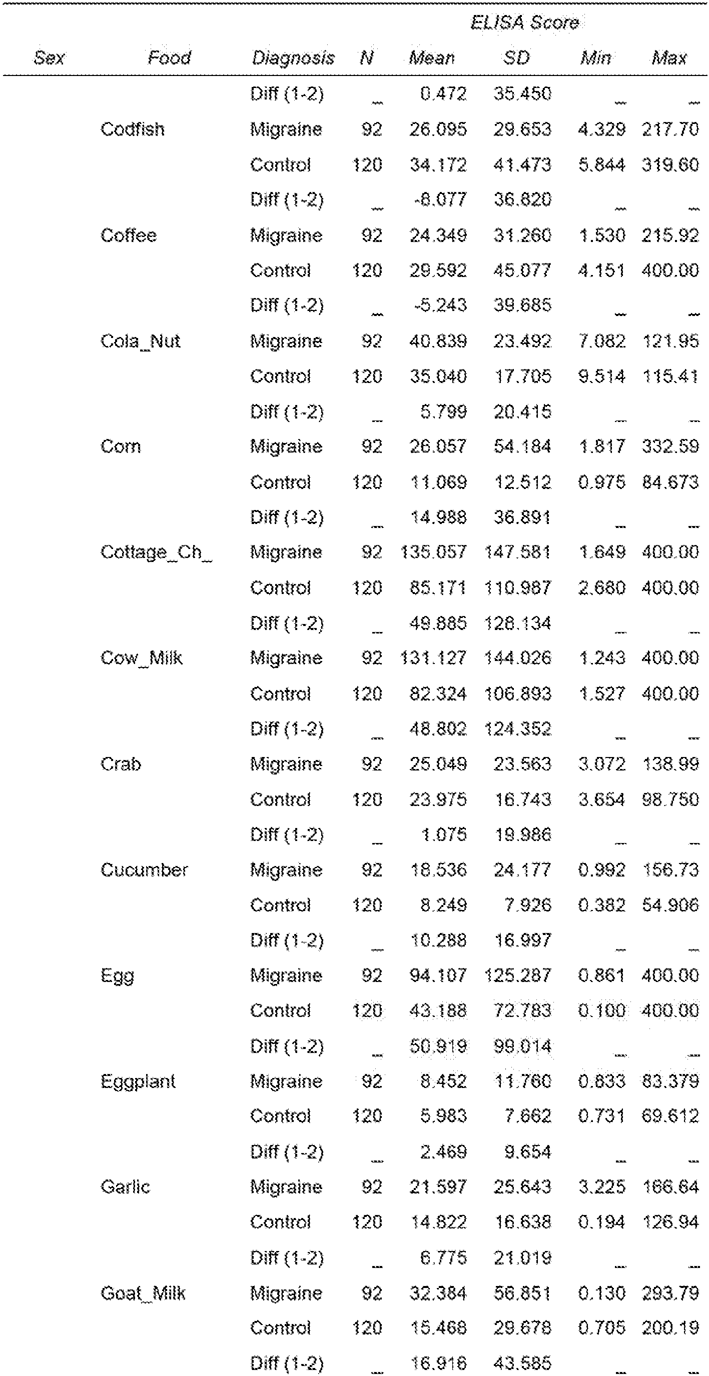

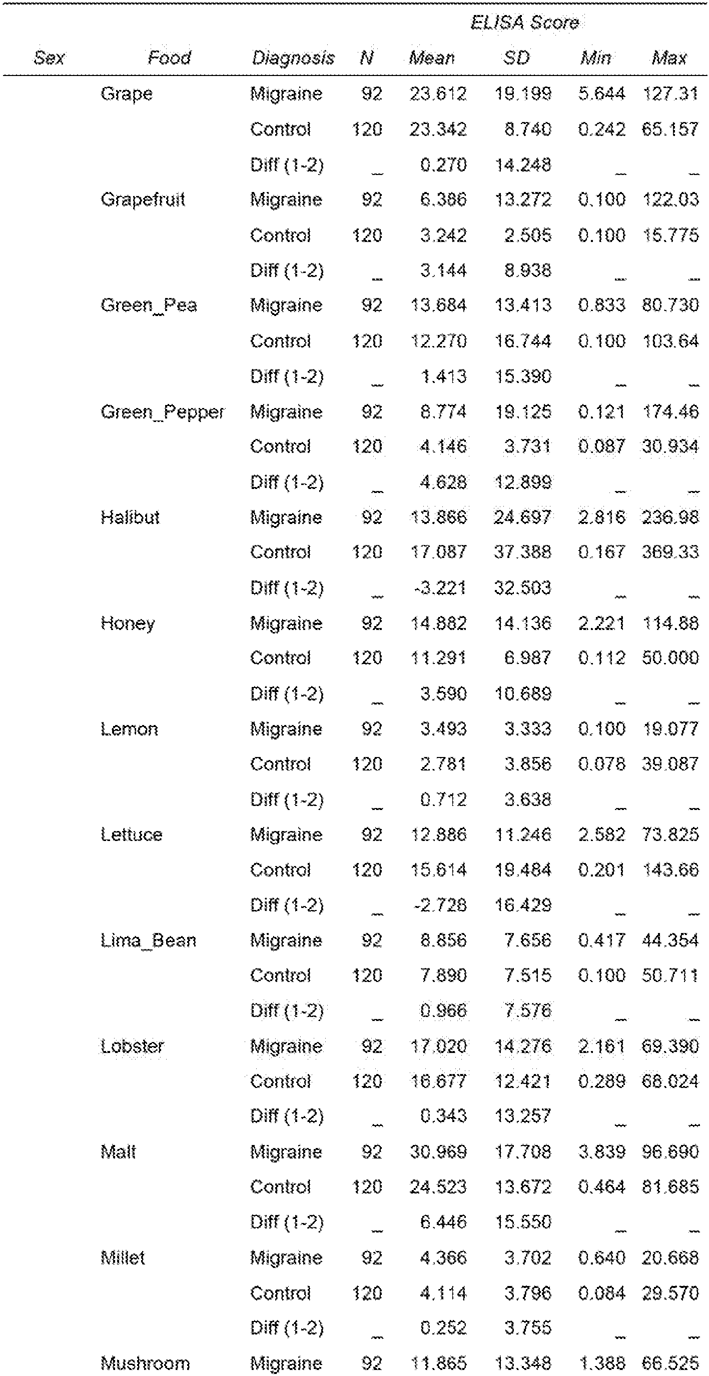

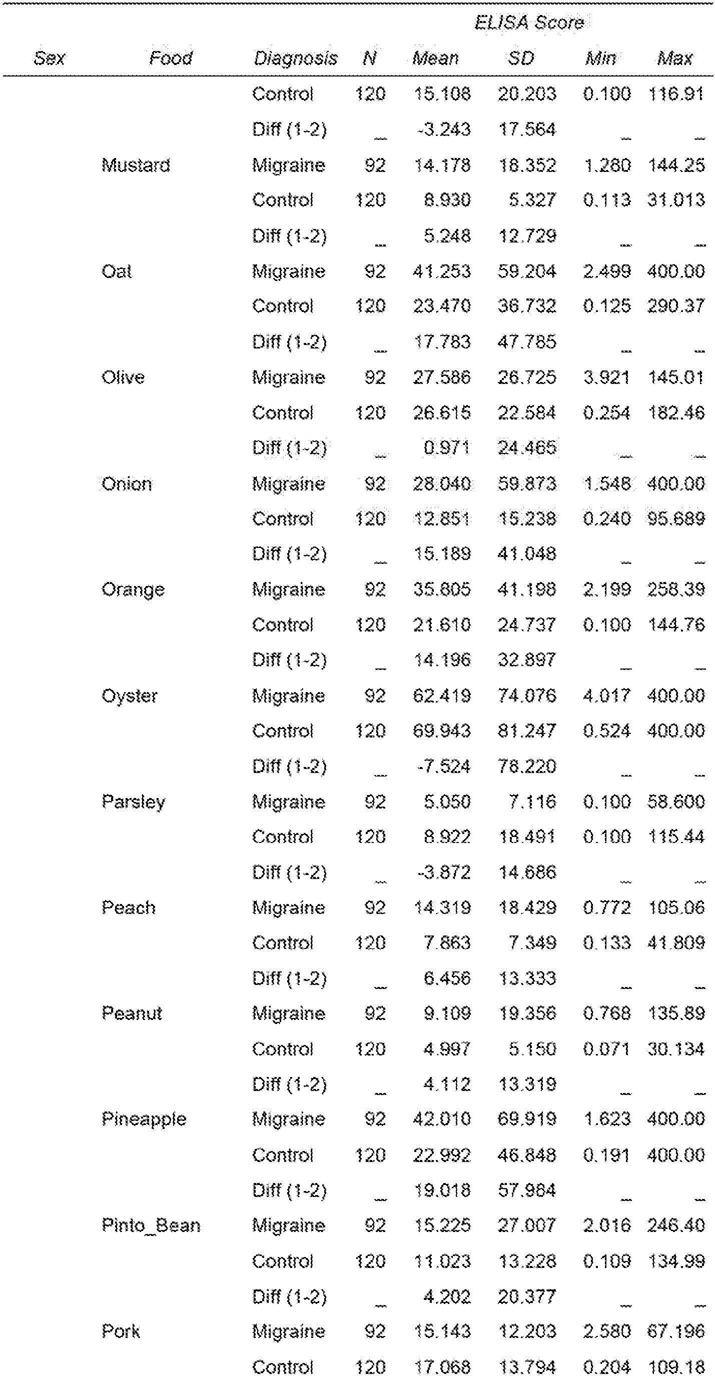

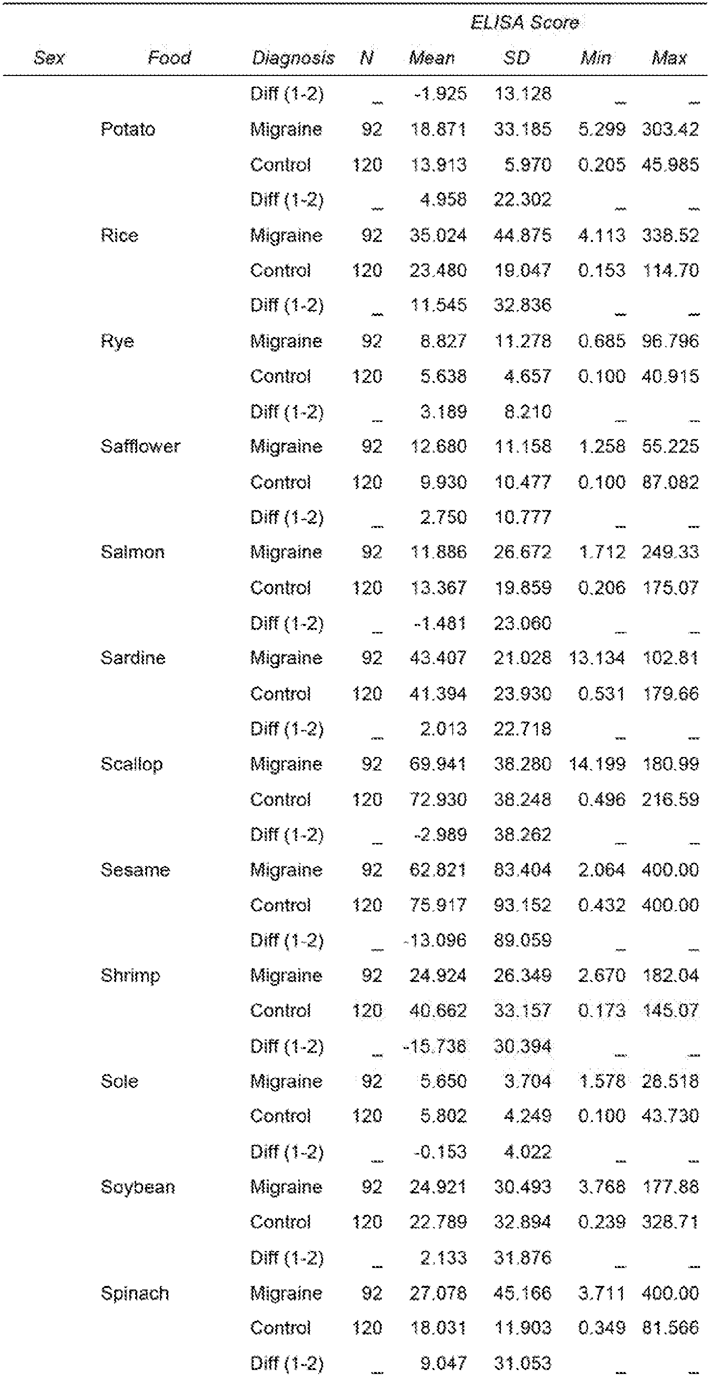

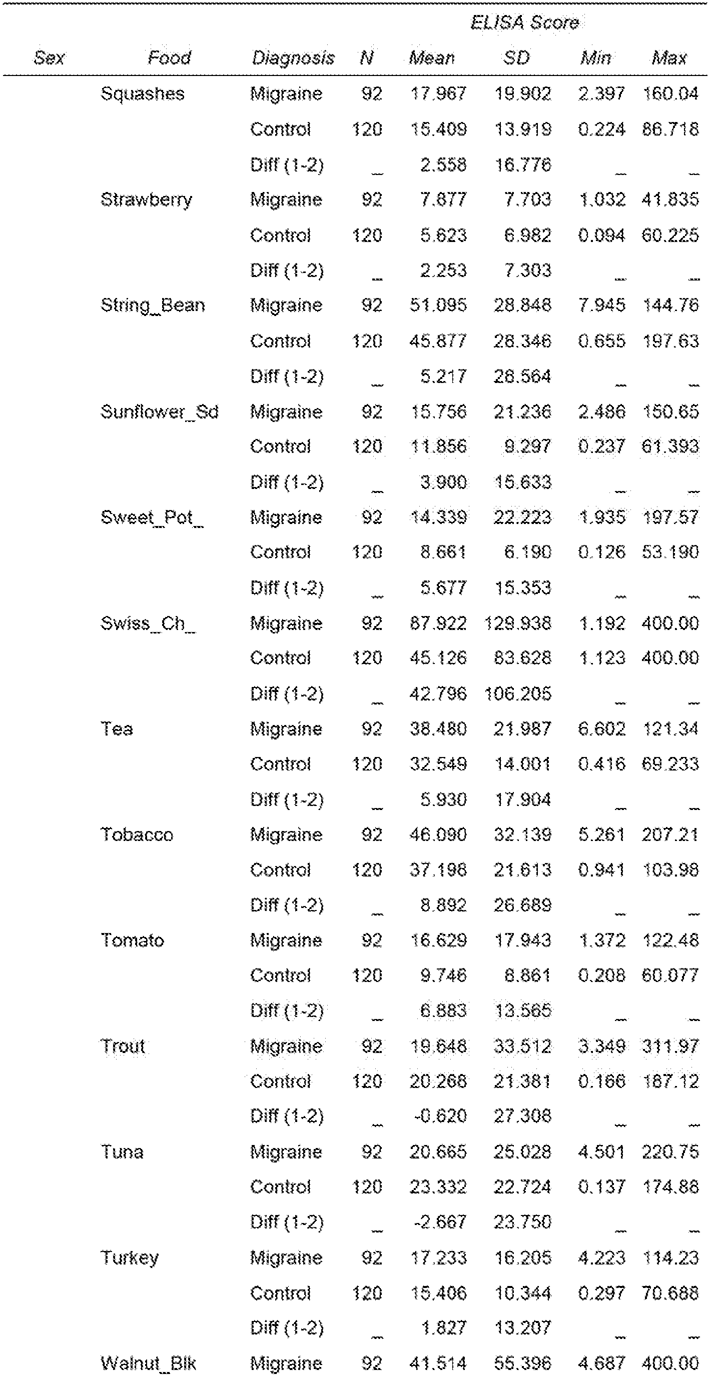

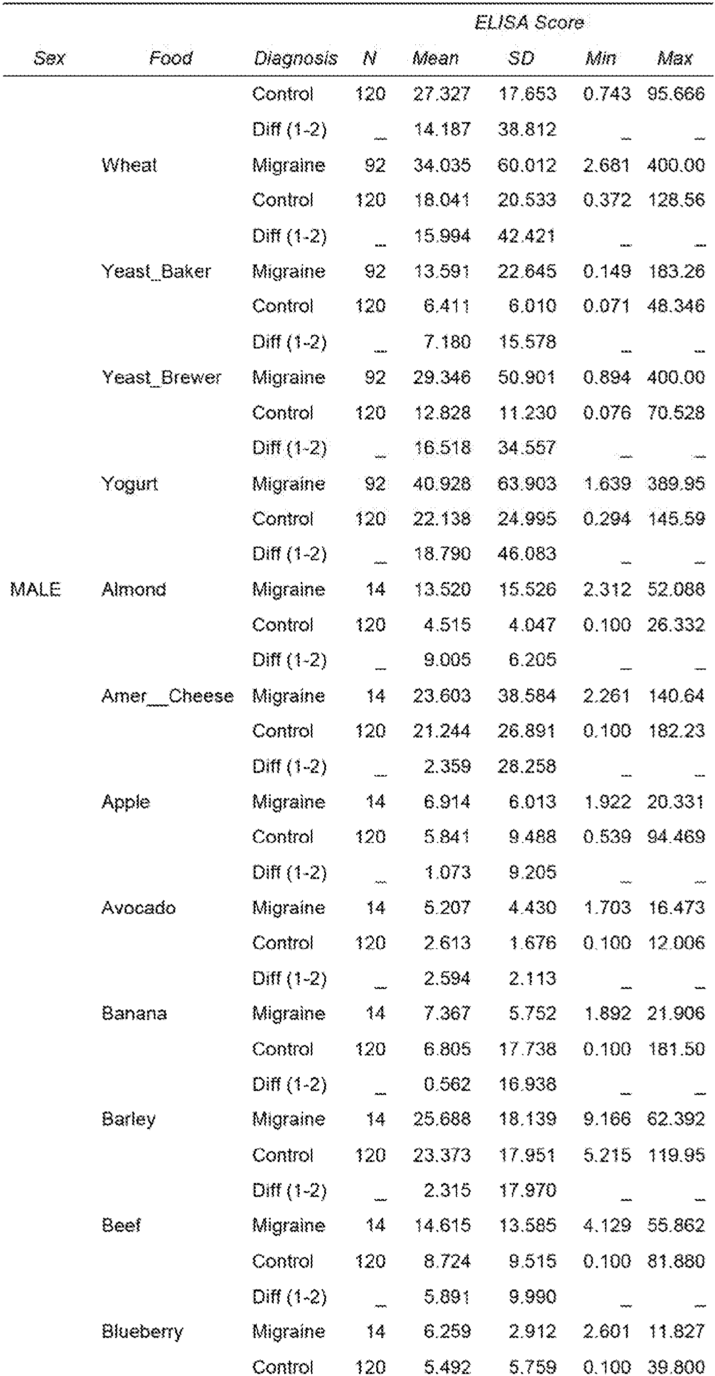

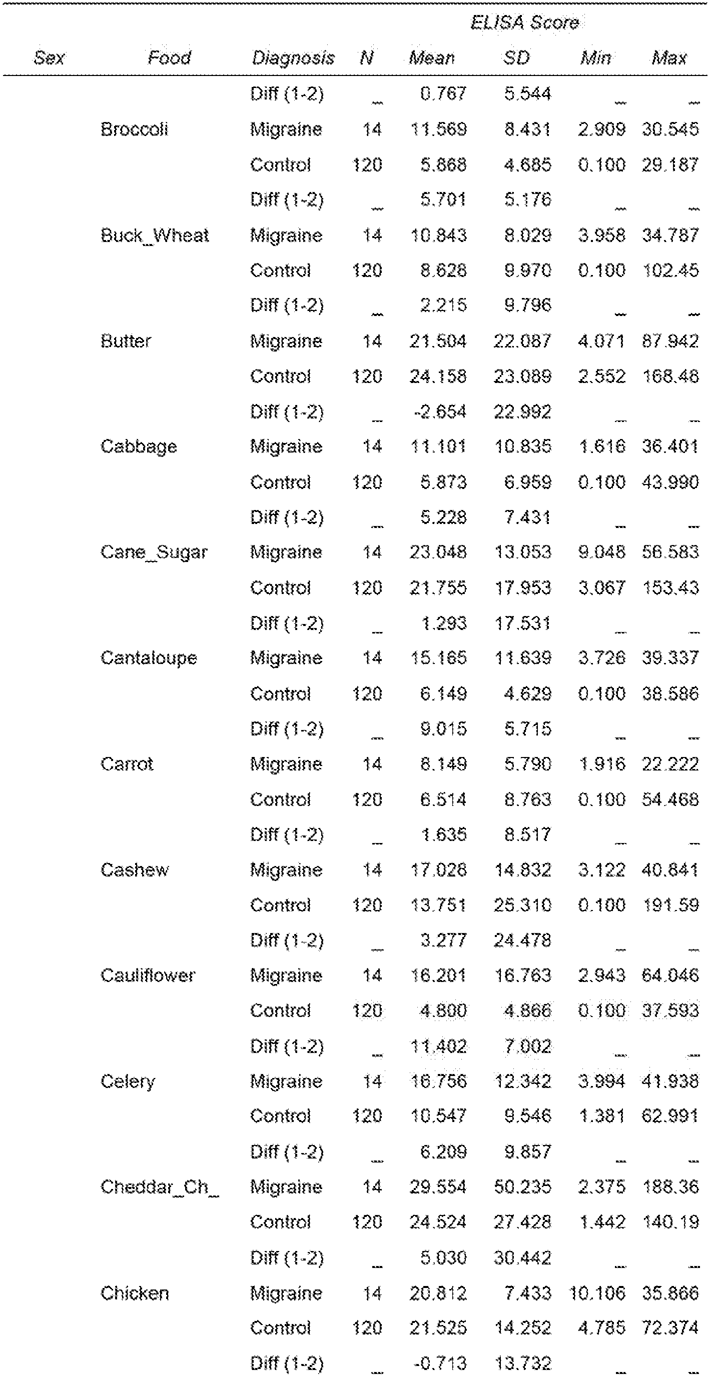

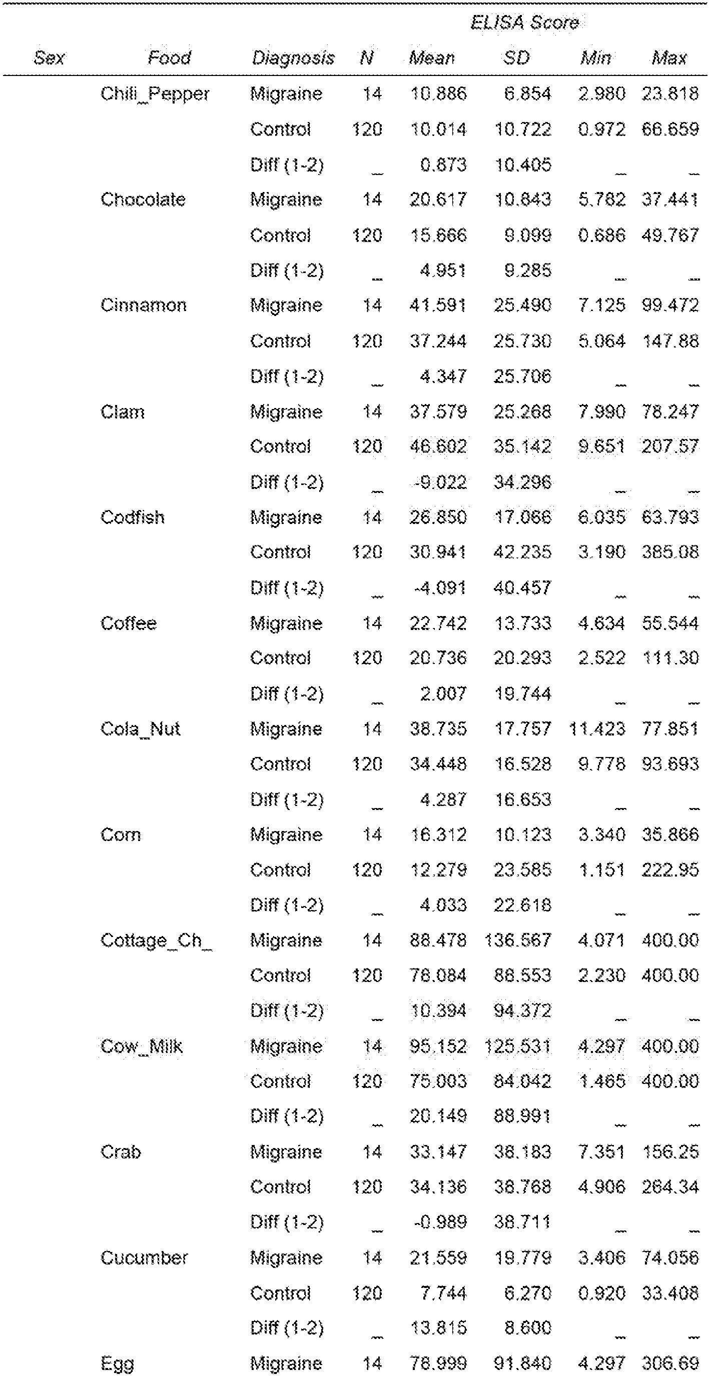

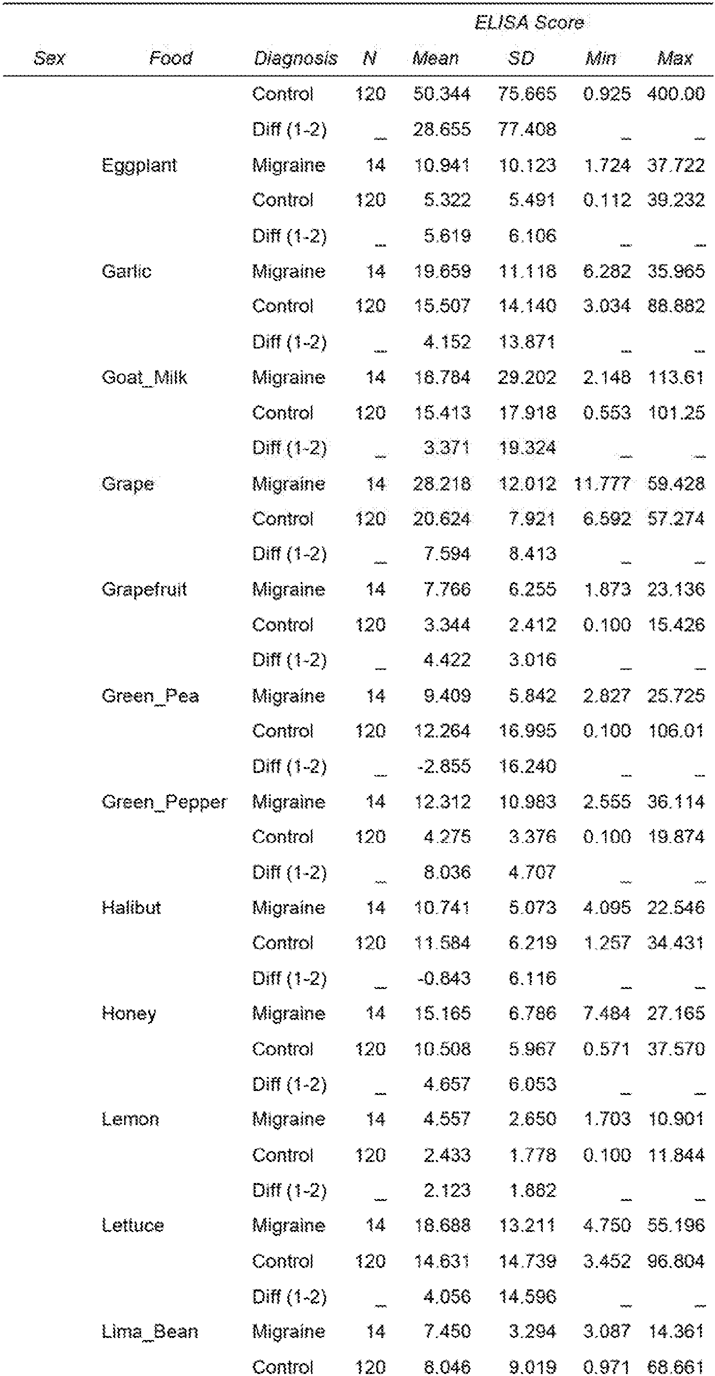

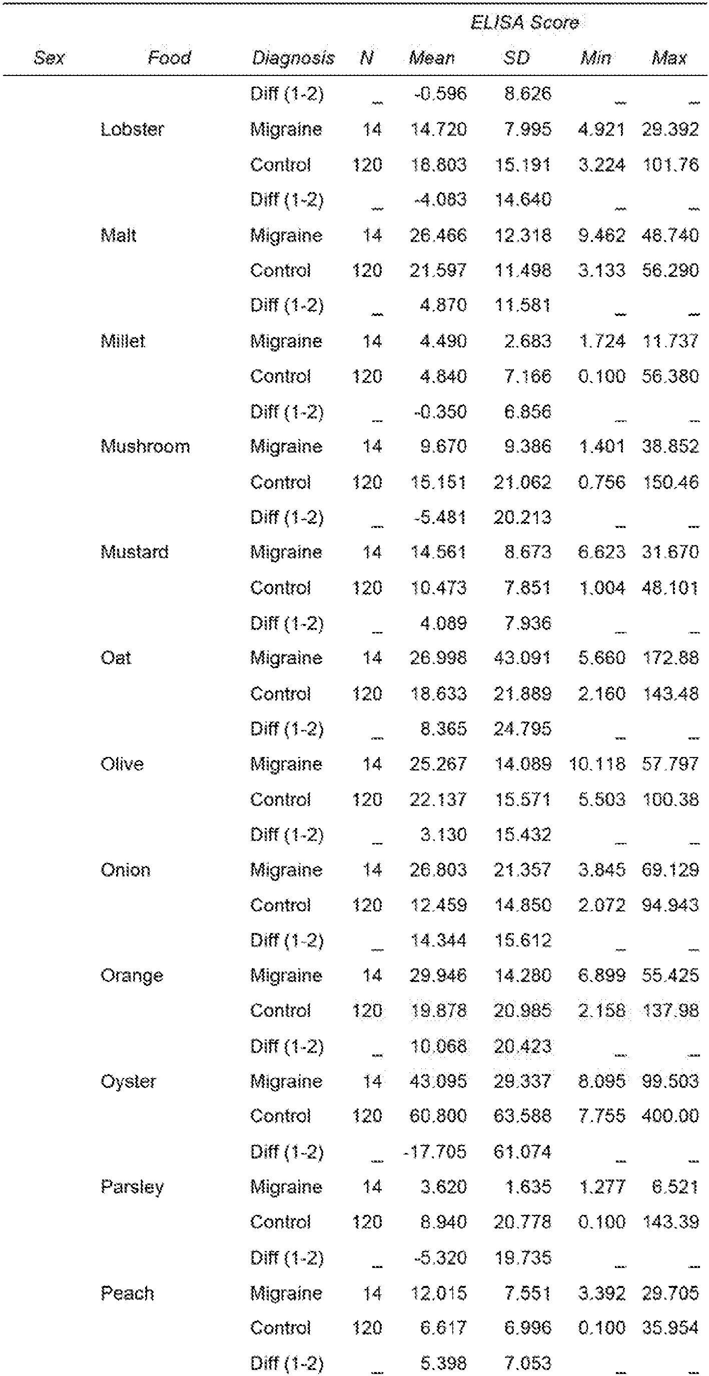

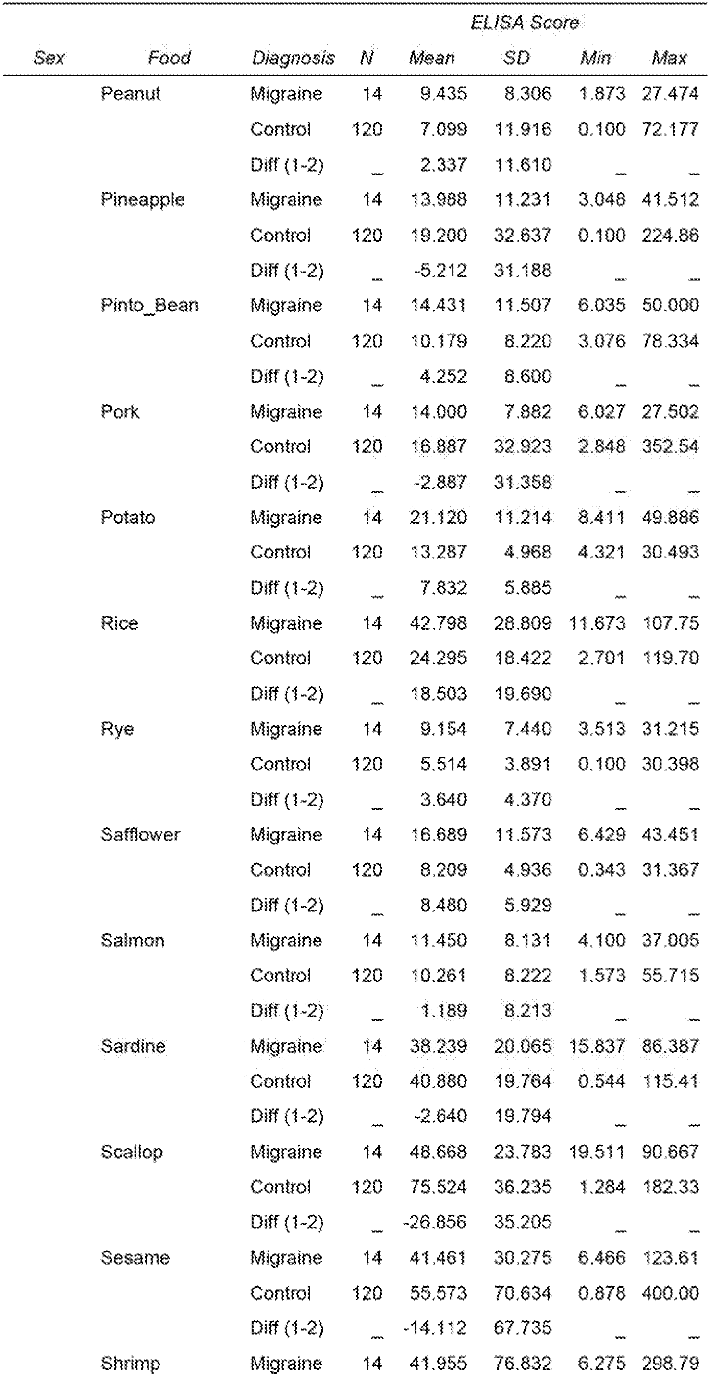

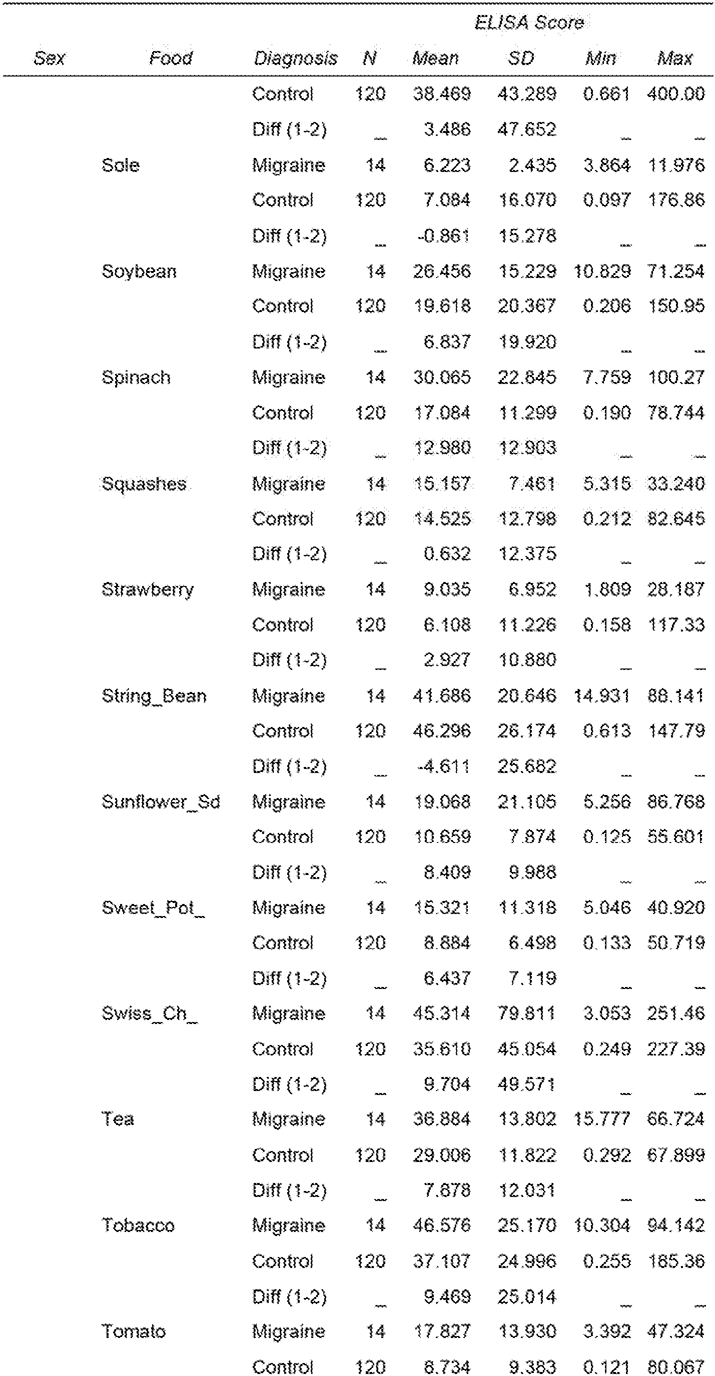

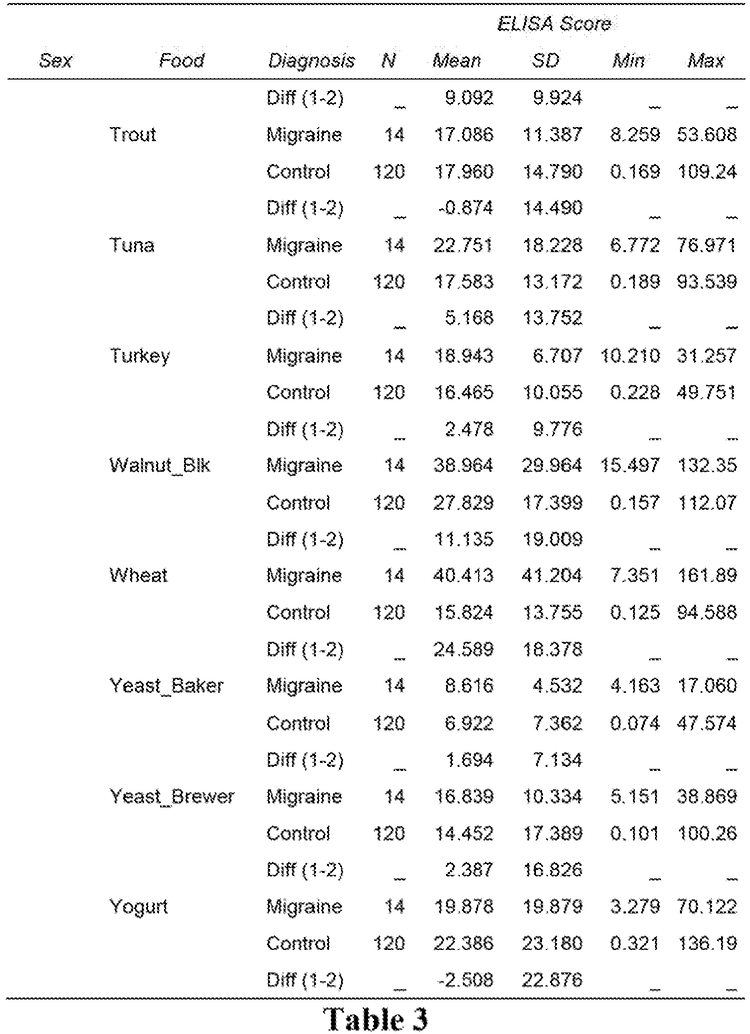

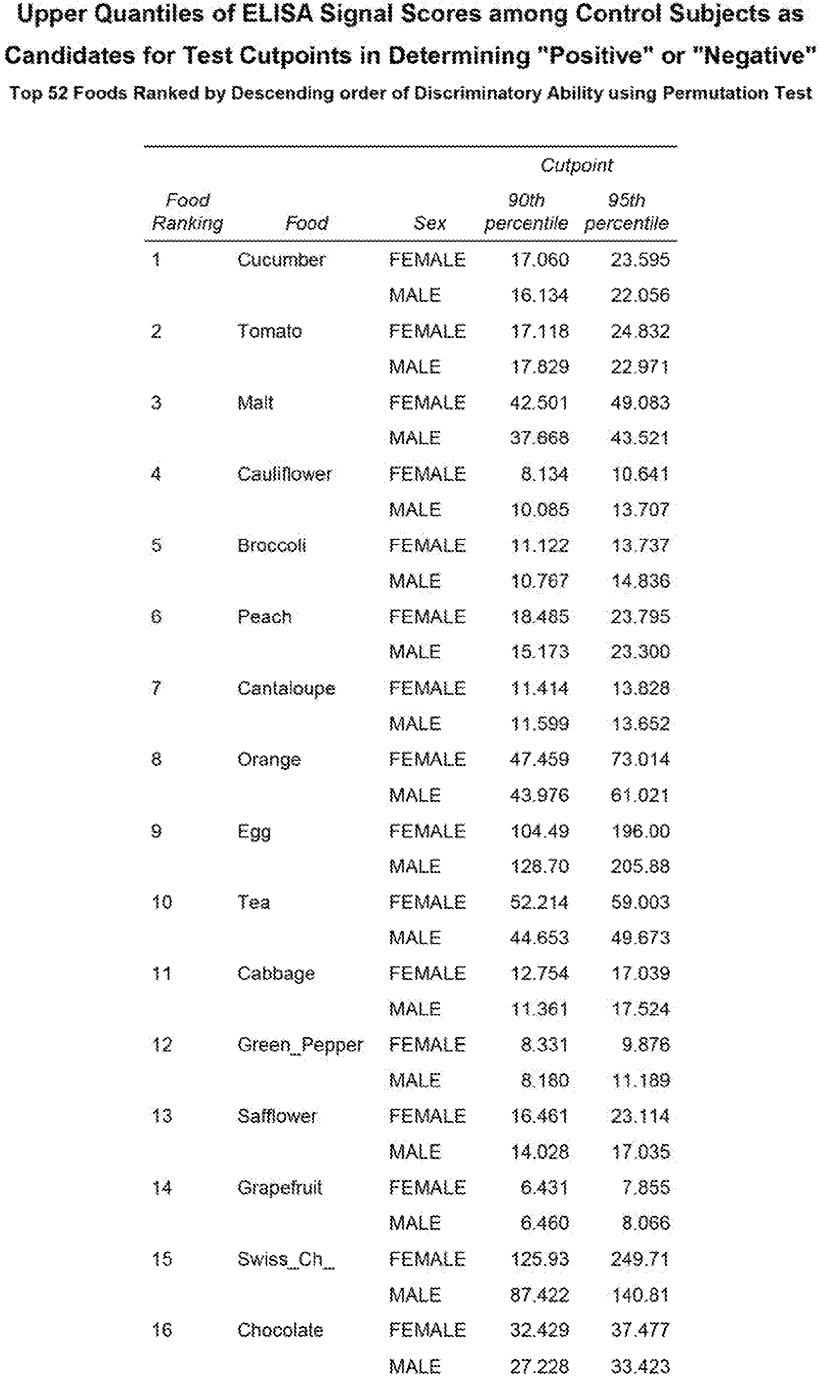

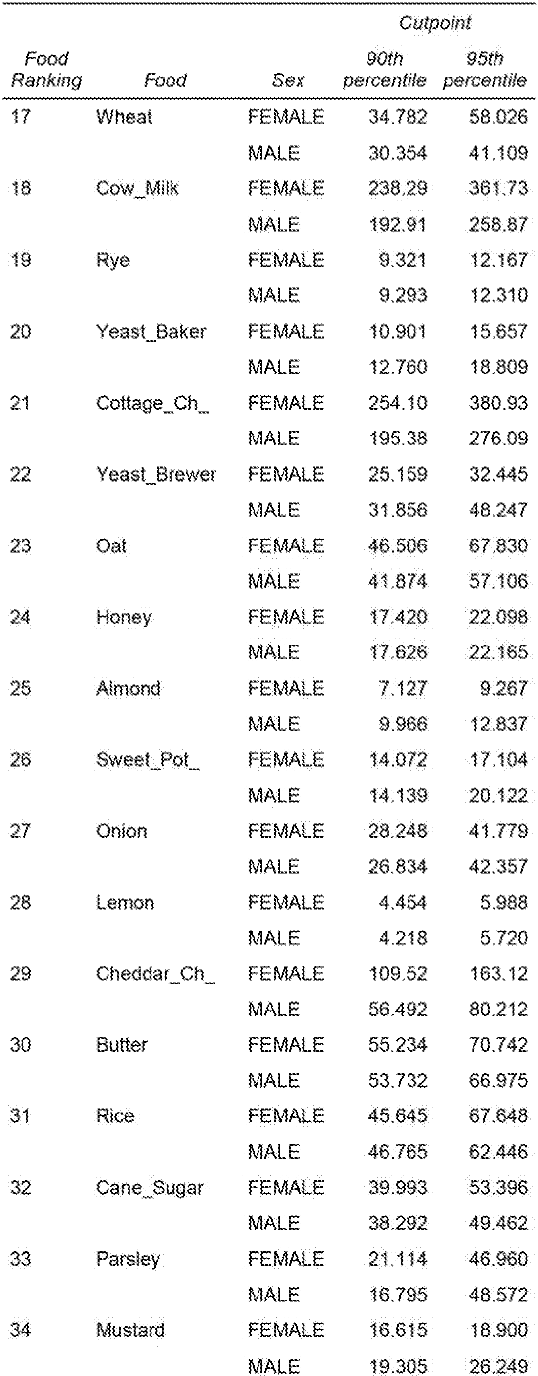

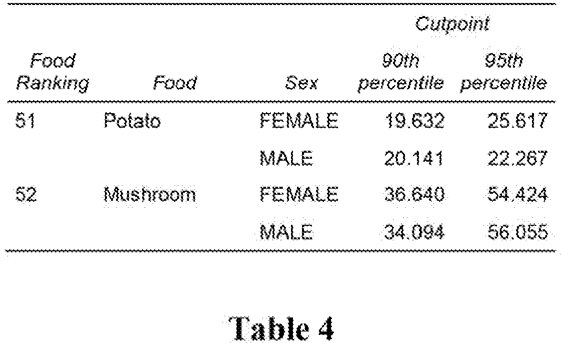

## Patentansprüche

1. Testpanel zum Testen auf Lebensmittelunverträglichkeit bei einem Patienten, bei dem Migräne diagnostiziert wurde oder bei dem der Verdacht besteht dass er diese aufweist, wobei das Testpanel Folgendes umfasst:
eine Vielzahl von unterschiedlichen Lebensmittelzubereitungen, wobei jede Lebensmittelzubereitung unabhängig an einen einzeln adressierbaren festen Träger gekoppelt ist;
wobei die Vielzahl von unterschiedlichen Lebensmittelzubereitungen aus Folgendem besteht: Gurke, Tomate, Malz, Blumenkohl, Brokkoli, Pfirsich, Cantalupe-Melone, Orange, Ei, Tee, Kohl, grünem Pfeffer, Saflor, Grapefruit, Schweizer Käse, Schokolade, Weizen, Kuhmilch, Roggen, Backhefe, Hüttenkäse, Bierhefe, Hafer, Honig, Mandeln, Süßkartoffeln, Zwiebel, Zitrone, Cheddar-Käse, Butter, Reis, Rohrzucker, Petersilie, Senf, Tabak, Ziegenmilch, amerikanischem Käse, Joghurt, Aubergine, Schwarznuss, Spinat, Kolanuss, Avocado, Mais, Knoblauch, Ananas, Erdbeere, Sonnenblumenkernen, Buchweizen, Rindfleisch, Kartoffeln und Pilzen.

2. Testpanel nach Anspruch 1, wobei jede der unterschiedlichen Lebensmittelzubereitungen einen rohen gefilterten wässrigen Extrakt oder einen verarbeiteten wässrigen Extrakt umfasst.

3. Testpanel nach Anspruch 1 oder Anspruch 2, wobei der feste Träger ein Well einer Multiwellplatte, ein Bead, ein elektrischer Sensor, ein chemischer Sensor, ein Mikrochip oder ein adsorptiver Film ist.

4. In-vitro-Verfahren zum Testen einer Lebensmittelunverträglichkeit bei Patienten, bei denen Migräne diagnostiziert wurde oder bei denen der Verdacht besteht dass sie diese aufweisen, umfassend:
Inkontaktbringen einer Lebensmittelzubereitung, wobei die Lebensmittelzubereitung mindestens eine Komponente aufweist, mit einer Körperflüssigkeit eines Patienten, bei dem Migräne diagnostiziert wurde oder bei dem der Verdacht besteht dass er diese aufweist, wobei die Körperflüssigkeit mindestens ein Immunglobulin umfasst, wobei die Körperflüssigkeit mit einer Geschlechtsidentifikation verbunden ist und wobei das Inkontaktbringen unter Bedingungen durchgeführt wird, die es mindestens einem Teil des Immunglobulins ermöglichen, sich an die mindestens eine Komponente zu binden;
Messen des Teils des Immunglobulins, der an die mindestens eine Komponente der Lebensmittelzubereitung gebunden ist, um ein Signal zu erhalten;
Vergleichen des Signals mit einem geschlechtsstratifizierten Referenzwert für die Lebensmittelzubereitung unter Verwendung der Geschlechtsidentifikation, um ein Ergebnis zu erhalten; und
Aktualisieren oder Generieren eines Berichts unter Verwendung des Ergebnisses,
wobei der Schritt des Inkontaktbringens einer Lebensmittelzubereitung mit einem Multiplex-Assay durchgeführt wird, das eine Vielzahl von unterschiedlichen Lebensmittelzubereitungen umfasst, und
wobei die Vielzahl von unterschiedlichen Lebensmittelzubereitungen aus Folgendem besteht: Gurke, Tomate, Malz, Blumenkohl, Brokkoli, Pfirsich, Cantalupe-Melone, Orange, Ei, Tee, Kohl, grünem Pfeffer, Saflor, Grapefruit, Schweizer Käse, Schokolade, Weizen, Kuhmilch, Roggen, Backhefe, Hüttenkäse, Bierhefe, Hafer, Honig, Mandeln, Süßkartoffeln, Zwiebel, Zitrone, Cheddar-Käse, Butter, Reis, Rohrzucker, Petersilie, Senf, Tabak, Ziegenmilch, amerikanischem Käse, Joghurt, Aubergine, Schwarznuss, Spinat, Kolanuss, Avocado, Mais, Knoblauch, Ananas, Erdbeere, Sonnenblumenkernen, Buchweizen, Rindfleisch, Kartoffeln und Pilzen.

5. Verfahren nach Anspruch 4, wobei die Körperflüssigkeit des Patienten Vollblut, Plasma, Serum, Speichel oder eine Stuhlsuspension umfasst.

6. Verfahren nach Anspruch 4 oder Anspruch 5, wobei der geschlechtsstratifizierten Referenzwert für jede der Lebensmittelzubereitungen der 90ste Perzentilrang oder höher von Signalen ist, die durch das Inkontaktbringen von Körperflüssigkeit von einer Kontrollgruppe von Subjekten, bei denen keine Migräne diagnostiziert wurde oder bei denen kein Verdacht besteht dass sie diese aufweisen, mit der Lebensmittelzubereitung erhalten werden.

## Revendications

1. Panel de tests permettant de tester une intolérance alimentaire chez un patient chez lequel des céphalées migraineuses ont été diagnostiquées ou sont suspectées, le panel de tests comprenant :
une pluralité de préparations alimentaires distinctes, chaque préparation alimentaire étant couplée indépendamment à un support solide adressable individuellement ;
ladite pluralité de préparations alimentaires distinctes étant constituée de concombre, tomate, malt, chou-fleur, brocoli, pêche, cantaloup, orange, oeuf, thé, chou, poivron vert, carthame, pamplemousse, fromage suisse, chocolat, blé, lait de vache, seigle, levure de boulanger, fromage cottage, levure de bière, avoine, miel, amande, patate douce, oignon, citron, fromage cheddar, beurre, riz, sucre de canne, persil, moutarde, tabac, lait de chèvre, fromage américain, yaourt, aubergine, noix noire, épinards, noix de cola, avocat, maïs, ail, ananas, fraise, graine de tournesol, sarrasin, boeuf, pomme de terre et champignon.

2. Panel de tests selon la revendication 1, chacune des préparations alimentaires distinctes comprenant un extrait aqueux filtré brut ou un extrait aqueux traité.

3. Panel de tests selon la revendication 1 ou la revendication 2, ledit support solide étant un puits d'une plaque multipuits, une bille, un capteur électrique, un capteur chimique, une micropuce ou un film adsorbant.

4. Procédé *in vitro* de test d'intolérance alimentaire chez des patients chez lesquels des céphalées migraineuses ont été diagnostiquées ou sont suspectées, comprenant :
la mise en contact d'une préparation alimentaire, ladite préparation alimentaire comportant au moins un constituant, avec un fluide corporel d'un patient chez lequel des céphalées migraineuses ont été diagnostiquées ou sont suspectées, ledit fluide corporel comprenant au moins une immunoglobuline, ledit fluide corporel étant associé à une identification de genre, et ladite mise en contact étant effectuée dans des conditions qui permettent à au moins une partie de l'immunoglobuline de se lier audit au moins un constituant ;
la mesure de la partie de l'immunoglobuline liée audit au moins un constituant de la préparation alimentaire pour obtenir un signal ;
la comparaison du signal à une valeur de référence stratifiée par genre pour la préparation alimentaire en utilisant l'identification de genre pour obtenir un résultat ; et
la mise à jour ou la génération d'un rapport en utilisant le résultat,
ladite mise en contact d'une préparation alimentaire étant réalisée avec un dosage multiplex comprenant une pluralité de préparations alimentaires distinctes ; et
ladite pluralité de préparations alimentaires distinctes étant constituée de concombre, tomate, malt, chou-fleur, brocoli, pêche, cantaloup, orange, oeuf, thé, chou, poivron vert, carthame, pamplemousse, fromage suisse, chocolat, blé, lait de vache, seigle, levure de boulanger, fromage cottage, levure de bière, avoine, miel, amande, patate douce, oignon, citron, fromage cheddar, beurre, riz, sucre de canne, persil, moutarde, tabac, lait de chèvre, fromage américain, yaourt, aubergine, noix noire, épinards, noix de cola, avocat, maïs, ail, ananas, fraise, graine de tournesol, sarrasin, boeuf, pomme de terre et champignon.

5. Procédé selon la revendication 4, ledit fluide corporel du patient comprenant du sang total, du plasma, du sérum, de la salive ou une suspension fécale.

6. Procédé selon la revendication 4 ou la revendication 5, ladite valeur de référence stratifiée selon le genre pour chacune des préparations alimentaires étant le rang du 90e percentile, ou plus, des signaux obtenus en mettant en contact un fluide corporel provenant d'un groupe témoin de sujets chez lesquels des céphalées migraineuses n'ont pas été diagnostiquées ou ne sont pas suspectées avec la préparation alimentaire.
